(19) Europäisches Patentamt / European Patent Office / Office européen des brevets

(11) **EP 4 199 871 B1**

(12) **EUROPEAN PATENT SPECIFICATION**

(45) Date of publication and mention
of the grant of the patent:
**20.11.2024 Bulletin 2024/47**

(21) Application number: **21749317.0**

(22) Date of filing: **26.07.2021**

(51) International Patent Classification (IPC):
*A61F 13/02* (2024.01)   *A61F 13/05* (2024.01)
*A61M 1/00* (2006.01)

(52) Cooperative Patent Classification (CPC):
**A61F 13/05; A61F 13/00987; A61F 13/0226;
A61M 1/90; A61M 1/915; B29C 44/0407;
B29C 44/5663; B29C 44/58;** B29K 2075/00;
B29K 2105/045; B29L 2031/753

(86) International application number:
**PCT/IB2021/056745**

(87) International publication number:
**WO 2022/038436 (24.02.2022 Gazette 2022/08)**

(54) **WOUND INTERFACE SYSTEMS WITH INTEGRAL CONTACT SURFACES**

WUNDSCHNITTSTELLENSYSTEME MIT INTEGRIERTEN KONTAKTFLÄCHEN

SYSTÈMES D'INTERFACE DE PLAIE À SURFACES DE CONTACT INTÉGRÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.08.2020 US 202063068524 P**

(43) Date of publication of application:
**28.06.2023 Bulletin 2023/26**

(73) Proprietor: **KCI Manufacturing Unlimited
Company
Athlone, Co. Westmeath, N37XF22 (IE)**

(72) Inventors:
• **JONIETZ, Bradley
Dublin Road Athlone, Co. Westmeath N37 XF22
(IE)**
• **CHILTON III, Robert
Dublin Road Athlone, Co. Westmeath N37 XF22
(IE)**

(74) Representative: **Simmons & Simmons
City Point
One Ropemaker Street
London EC2Y 9SS (GB)**

(56) References cited:
WO-A1-2018/226707      WO-A1-2020/081322
WO-A2-2012/082716      US-A1- 2018 289 558
US-A1- 2019 053 819      US-A1- 2020 085 629

**Description**

**TECHNICAL FIELD**

The invention set forth in the appended claims relates generally to tissue treatment systems and more particularly, but without limitation, to dressings for tissue treatment and methods for tissue treatment with negative pressure.

**BACKGROUND**

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

WO 2020/081322 A1 concerns a peel and place dressing having a closed-cell contact layer.

While the clinical benefits of negative-pressure therapy and/or instillation therapy are widely known, improvements to therapy systems, components, and processes may benefit healthcare providers and patients.

**BRIEF SUMMARY**

Dressings for treating tissue in a negative-pressure therapy environment are set forth in the appended claims. Illustrative embodiments are also provided to enable a person skilled in the art to make and use the claimed subject matter.

For example, in some embodiments, a dressing for treating a tissue site with negative pressure may include a foam member having a felted portion and a non-felted portion. The felted portion may extend a depth into a surface of the foam member. The dressing may also include a sealing layer with a treatment aperture and a first plurality of perforations around the treatment aperture. The felted portion may be configured to contact the tissue site through the treatment aperture. A second plurality of perforations may be formed through the felted portion, and at least one of the second plurality of perforations may be exposed to the tissue site through the treatment aperture. The dressing may also include a cover. The cover may include a film and an adhesive. The film may be disposed over the foam member and coupled to the sealing layer around the foam member. The adhesive may be disposed adjacent to the second plurality of perforations.

According to example embodiments, the foam member may include an open-cell foam. Herein, 1 lb/ft$^3$ = 16.0185 kg/m$^3$, 1 pore per inch = 1 pore per 25.4 mm, and 1 pound per square inch = 6.895 kPa. In illustrative embodiments, the non-felted portion may have a density in a range of about 1.3 lb/ft$^3$ to about 1.6 lb/ft$^3$. In some embodiments, the felted portion may have a density in a range of about 2.6 lb/ft$^3$ to about 16 lb/ft$^3$. In example embodiments, the non-felted portion may have an average pore size in a range of about 400 micrometers to about 600 micrometers. In some embodiments, the felted portion may have an average pore size in a range of about 50 micrometers to about 300 micrometers. According to illustrative embodiments, the non-felted portion may have a free volume of about 90%. In example embodiments, the felted portion may have a free volume in a range of about 9% to about 45%. In some embodiments, the non-felted portion may have an average porosity in a range of about 40 pores per inch to about 50 pores per inch. According to some embodiments, the felted portion may have an average porosity in a range of about 80 pores per inch to about 500 pores per inch. In illustrative embodiments, the non-felted portion may have a 25% compression load deflection of about 0.35 pounds per square inch. Some examples of the dressing may have a felted portion with a 25% compression load deflection of about 0.7 pounds per square inch to about 3.5 pounds per square inch. According to some examples, the non-felted portion may have a 65% compression load deflection of about 0.43 pounds per square inch. In some examples, the felted portion may have a 65% compression load deflection in a range of about 0.86 pounds per square inch to about 4.3 pounds per square inch.

According to illustrative examples, each perforation of the second plurality of perforations may be a slot formed through the felted portion. In some examples, the slot may have a length in a range of about 1 millimeter to about 5 millimeters and a width in a range of about 0.4 millimeters to about 1 millimeter. In some embodiments, the slots may be distributed across the felted portion in a uniform pattern of parallel rows and columns. According to some embodiments, the slots may be spaced about 3 millimeters on center. In illustrative embodiments, each of the rows may be spaced about 3 millimeters on center. According to example embodiments, the slots in adjacent rows may be offset.

In some embodiments, the dressing may further include a third plurality of perforations formed through the felted portion. In some illustrative examples, each perforation of the third plurality of perforations may be a slot formed through the felted portion. According to example embodiments, each perforation of the second plurality of perforations may be parallel to a first direction, and each perforation of the third plurality of perforations may be parallel to a second direction. In some examples, the second plurality of perforations may be arranged in a first pattern of rows, and the third plurality of perforations may be arranged in a second pattern of rows. In illustrative embodiments, the first direction may be orthogonal to the second direction. In illustrative examples, the foam member may include an edge, and the first direction may be parallel to the edge. According to example embodiments, and the first direction may be rotated an angle $\theta$ in a range of about 0° to about 90° with respect to the edge. In some examples, $\theta$ may be about 45°. In some embodiments, the foam member may include an edge, and the second direction may be rotated an angle $(p$ in a range of about 0° to about 180° with respect to the edge. In example embodiments, the second direction may be orthogonal to the edge. In illustrative embodiments, $\varphi$ may be about 135°. According to illustrative examples, the first pattern of rows may be staggered a third angle $\beta$, wherein $\beta$ may be about 45°. According to some examples, the second pattern of rows may be staggered a fourth angle y, wherein y may be about 135°.

Further, some example embodiments may include a dressing for treating a tissue site with negative pressure. The dressing may include a cover having an adhesive and a felted foam member having a felted surface. The felted foam member may have a first plurality of perforations formed through the felted foam member. The dressing may also include a perforated gel layer having a treatment aperture. The cover, the felted foam member, and the perforated gel layer may be assembled in a stacked relationship with the cover and the perforated gel enclosing the felted foam member. The felted surface may be at least partially exposed through the treatment aperture. At least some of the adhesive may be exposed through the perforated gel layer around the treatment aperture.

According to illustrative embodiments, the felted foam member may include an open-cell foam. For example, the felted foam member may include a reticulated polyurethane foam. In some embodiments, the felted foam member may have a density in a range of about 2.6 lb/ft$^3$ to about 16 lb/ft$^3$. In example embodiments, the felted foam member may have an average pore size in a range of about 50 micrometers to about 300 micrometers. In illustrative embodiments, the felted foam member may have a free volume in a range of about 9% to about 45%. According to some embodiments, the felted foam member may have an average porosity in a range of about 80 pores per inch to about 500 pores per inch. In some examples, the felted foam member may have a 25% compression load deflection in a range of about 0.7 pounds per square inch to about 3.5 pounds per square inch. In some example embodiments, the felted foam member may have a 65% compression load deflection of about 0.86 pounds per square inch to about 4.3 pounds per square inch.

According to illustrative examples, the dressing may also include an open-cell foam member disposed between the felted foam member and the cover. In some examples, the open-cell foam member may include a reticulated open-cell polyurethane foam having a density in a range of about 1.3 lb/ft$^3$ to about 1.6 lb/ft$^3$, an average pore size in a range of about 400 micrometers to about 600 micrometers, a free volume of about 90%, an average porosity in a range of about 40 pores per inch to about 50 pores per inch, a 25% compression load deflection of about 0.32 pounds per square inch, and a 65% compression load deflection of about 0.43 pounds per square inch. In some embodiments, each of the first plurality of perforations may be a slot formed through the felted foam member. In illustrative embodiments, the slot may have a length in a range of about 1 millimeter to about 5 millimeters. According to some examples, the slots may be arranged in a uniform pattern of parallel rows and columns. In example embodiments, the slots may be spaced about 3 millimeters on center. In illustrative examples, the slots in adjacent rows may be offset.

A system for treating a tissue site with negative pressure is also described herein, wherein some example embodiments include a dressing configured to be placed adjacent to the tissue site. The dressing may include a sealing layer with a central aperture and a first plurality of perforations around the central aperture. The dressing may also include a felted foam member having a second plurality of perforations extending through the felted foam member. At least a portion of the felted foam member may be exposed through the central aperture. A film may be disposed over the felted foam member and the sealing layer. The film may be coupled to the sealing layer around the felted foam member. The system may also include a negative-pressure source configured to provide negative pressure to the tissue site through the felted foam member.

In some examples, the felted foam member may include an open-cell reticulated polyurethane foam. According to illustrative embodiments, the felted foam member may have a density in a range of about 2.6 lb/ft$^3$ to about 16 lb/ft$^3$, an average pore size in a range of about 50 micrometers to about 300 micrometers, a free volume in a range of about 9% to about 45%, an average porosity in a range of about 80 pore per inch to about 500 pores per inch, a 25% compression load deflection in a range of about 0.7 pounds per square inch to about 3.5 pounds per square inch, and a 65% compression load deflection in a range of about 0.86 pounds per square inch to about 4.3 pounds per square inch.

In example embodiments, the felted foam member may include an open-cell reticulated polyurethane foam. In some embodiments, the felted foam member may have a density in a range of about 3.4 lb/ft$^3$ to about 17 lb/ft$^3$, an average pore size in a range of about 40 micrometers to about 300 micrometers, an average porosity in a range of about 40 pores per inch to about 50 pores per inch, a 25% compression load deflection in a range of about 0.7 pounds per square inch to about 3.5 pounds per square inch, and a 65% compression load deflection in a range of about 1.2 pounds per square inch to about 6 pounds per square inch.

Some embodiments of the system may also include an open-cell foam member disposed between the felted foam member and the sealing layer. In example embodiments, the open-cell foam member may include a reticulated open-cell polyurethane foam having an average pore size in a range of about 400 micrometers to about 600 micrometers, an average porosity in a range of about 40 pores per inch to about 50 pores per inch, and a 25% compression load deflection of about 0.35 pounds per square inch. In some examples, the open-cell foam member may have a density in a range of about 1.3 lb/ft$^3$ to about 1.6 lb/ft$^3$, a free volume of about 90%, and a 65% compression load deflection of about 0.43 pounds per square inch. In example embodiments, the open-cell foam member may have a density in a range of about 1.7 lb/ft$^3$ to about 2.1 lb/ft$^3$ and a 65% compression load deflection of about 0.6 pounds per square inch.

According to some examples of the system, each of the second plurality of perforations may be a slot formed through the foam member. In some embodiments, the system may further include a third plurality of perforations formed through the felted foam member. In illustrative embodiments, each of the third plurality of perforations may be a slot formed through the felted foam member. In some examples, each of the second plurality of perforations may be parallel to a first direction, and each of the third plurality of perforations may be parallel to a second direction. In some embodiments, the second plurality of perforations may be arranged in a first pattern of rows, and the third plurality of perforations may be arranged in a second pattern of rows. According to illustrative embodiments, the first direction may be orthogonal to the second direction. In example embodiments, the felted foam member may include an edge, and the first direction may be parallel to the edge. In illustrative examples, the first direction may be rotated an angle $\theta$ in a range of about 0° to about 90° with respect to the edge. In some examples, $\theta$ may be about 45°. According to example embodiments, the second direction may be rotated an angle $\varphi$ in a range of about 0° to about 180° with respect to the edge. In illustrative embodiments, $\varphi$ may be about 135°. According to some examples of the system, the first pattern of rows may be staggered a third angle $\beta$, and the second pattern of rows may be staggered a fourth angle $y$. In some embodiments, $\beta$ may be about 45°. In example embodiments, y may be about 135°.

A method for manufacturing a dressing is also described, the method including the steps of placing a pre-polymer mixture and water into a mold to generate a reaction that produces a foam and an integral film on at least one surface of the foam, forming a first plurality of perforations in the integral film of the foam, providing a gel layer having a treatment aperture and a second plurality of perforations disposed around the treatment aperture, providing a cover with adhesive disposed on a side of the cover, and assembling the cover, the foam, and the gel layer in a stacked relationship. The cover and the gel layer may enclose the foam, the foam may be at least partially exposed through the treatment aperture, and at least some of the adhesive may be exposed through some of the second plurality of perforations.

According to some embodiments of the method, the mold may be an open tray, and the method may further include the step of skiving a top surface of the foam formed in the open tray. In example embodiments, the mold may include a bottom side having a first surface facing an interior of the mold. In illustrative embodiments, the first surface may be smooth. In some embodiments, each of the first plurality of perforations may include a slit formed through the integral foam. In some examples, each of the slits may have a length in a range of about 2 millimeters to about 5 millimeters. According to illustrative embodiments, the slits may be distributed across the integral film in a uniform pattern of parallel rows and columns. In some embodiments, the slits may be spaced about 3 millimeters on center. In example embodiments, each of the rows may be spaced about 3 millimeters on center. In illustrative embodiments, the slits in adjacent rows may be offset. According to example embodiments, the step of forming the first plurality of perforations may include using a blade to make the slits in the integral film.

According to illustrative embodiments, the mold may include a bottom surface and a plurality of projections on the bottom surface. In example embodiments, the first plurality of perforations may be formed by the plurality of projections. In some examples, each projection may have a length in a range of about 1 millimeter to about 5 millimeters. In illustrative examples, each projection may have a width in a range of about 0.4 millimeters to about 1 millimeter. In some embodiments, the projections may be distributed across the bottom surface in a uniform pattern of rows and columns. According to some examples, the projections may be spaced about 3 millimeters on center. In example embodiments, each of the rows may be spaced about 3 millimeters on center. According to illustrative examples, the projections in adjacent rows may be offset.

Other example embodiments may include a mold for forming a foam tissue interface. The mold may include a plurality of walls, a base portion, and a first plurality of projections formed on the base portion. The first plurality of projections may be arranged in a first pattern of rows. Each of the first plurality of projections may be parallel to a first direction. In some examples, the projections within each row of the first pattern of rows may be evenly distributed. In illustrative embodiments, the rows of the first pattern of rows may be evenly distributed. In example embodiments, the first pattern of rows may have a first pitch $P_1$ in a range of about 4 millimeters to about 6 millimeters. In some examples, each projection in each row of the first pattern of rows may be spaced a first distance $D_1$ from an adjacent projection. For example, $D_1$ may be in a range of about 3 millimeters to 5 millimeters. According to some embodiments, the first direction may be rotated a first angle $\theta$ in a range of about 0° to about 90° with respect to an edge of the base portion. In some examples, $\theta$ may be about 45°. In illustrative embodiments, each projection of the first plurality of projections may have a centroid incident to a reference line parallel to a third direction. In some embodiments, the third direction may be orthogonal to the first direction. According to some embodiments, the first pattern of rows may be staggered a third angle $\beta$. In some embodiments, $\beta$ may be about 45°. In some examples, each projection of the first plurality of projections may have a linear slot profile. In illustrative examples, each projection of the first plurality of projections may have a curved slot profile. According to some examples, each projection of the first plurality of projections may have a chevron slot shape.

Some example embodiments may include a second plurality of projections formed on the base portion. The second plurality of projections may be arranged in a second pattern of rows. Each of the second plurality of projections may be parallel to a second direction. In some embodiments, the rows of the second pattern may be evenly distributed. In example embodiments, the second pattern of rows may have a second pitch $P_2$ in a range of about 3 millimeters to about 6 millimeters. According to illustrative examples, each projection in each row of the second pattern of rows may be spaced a second distance $D_2$ from an adjacent projection. For example, $D_2$ may be in a range of about 3 millimeters to about 5 millimeters. In some embodiments, the first direction may be orthogonal to the second direction. In example embodiments, the second direction may be orthogonal to an edge of the base portion. In illustrative embodiments, the first direction may be parallel to the second direction. In some examples, the second direction may be rotated a second angle $(p$ in a second range of about 0° to about 180° with respect to an edge of the base portion. For example, $(p$ may be about 135°. In some examples, each projection of the second plurality of projections may have a centroid incident to a reference line in a fourth direction. In illustrative embodiments, the fourth direction may be parallel to the first direction.

According to some examples, each projection of the first plurality of projections may have a first centroid, each projection of the second plurality of projections may have a second centroid, and each of the first centroids may be aligned with one of the second centroids along a fourth direction. In some examples, each of the first centroids may be coincident with one of the second centroids. According to illustrative embodiments, the second pattern of rows may be staggered a fourth angle y. For example, y may be about 135°. In exemplary embodiments, each projection of the second plurality of projections may have a linear slot profile. In some examples, each projection of the second plurality of projections may have a curved slot profile. In illustrative examples, each projection of the second plurality of projections may have a chevron slot profile. In some examples, the base portion may include a margin without projections. For example, the margin may be between about 30% to about 80% of an area of the base portion. In illustrative embodiments, the plurality of walls may form a stadium shape. In some embodiments, the plurality of walls may have a profile formed of a rectangle with semicircles at opposite ends of the rectangle.

Objectives, advantages, and a preferred mode of making and using the claimed subject matter may be understood best by reference to the accompanying drawings in conjunction with the following detailed description of illustrative embodiments.

**BRIEF DESCRIPTION OF THE DRAWINGS**

Figure 1 is a block diagram of an example embodiment of a therapy system including an example dressing that can provide negative-pressure treatment and instillation treatment in accordance with this specification;

Figure 2A is an exploded view of an example of the dressing of Figure 1, illustrating additional details that may be associated with some embodiments in which a tissue interface that may be associated with the dressing includes more than one layer;

Figure 2B is an exploded view of an example of the dressing of Figure 1, illustrating additional details that may be associated with some embodiments in which a tissue interface that may be associated with the dressing includes more than one layer;

Figure 2C is an exploded view of an example of the dressing of Figure 1, illustrating additional details that may be associated with some embodiments in which a tissue interface that may be associated with the dressing includes more than one layer;

Figure 2D is an exploded view of an example of the dressing of Figure 1, illustrating additional details that may be associated with some embodiments in which a tissue interface that may be associated with the dressing includes more than one layer;

Figure 2E is an isometric view of an assembled example of the dressing of Figure 2C;

Figure 3 is a top view of the dressing in the example of Figure 2C, as assembled, illustrating additional details that may be associated with some embodiments;

Figure 4 is a bottom view of the dressing in the example of Figure 2C, as assembled, illustrating additional details that may be associated with some embodiments;

Figure 5 is an exploded view of another example of the dressing of Figure 1, illustrating additional details that may be associated with some embodiments;

Figure 6 is a bottom view of an example of a manifold that may be associated with any example embodiments of the dressing or the tissue interface, illustrating an example pattern of perforations that may be associated with some embodiments;

Figure 7 is a bottom view of another example of a manifold that may be associated with any example embodiments of the dressing or the tissue interface, illustrating another example of a pattern that may be associated with some embodiments of the perforations;

Figure 8 is a bottom view of another example of a manifold that may be associated with any example embodiments of the dressing or the tissue interface, illustrating another example pattern that may be associated with some illustrative embodiments of the perforations;

Figure 9 is a bottom view of another example of a manifold that may be associated with any example embodiments of the dressing or the tissue interface, illustrating an additional example of a pattern that can be associated with some embodiments of the perforations;

Figure 10 is a bottom view of another example of a manifold that may be associated with any example embodiments of the dressing or the tissue interface, illustrating additional embodiments of a pattern that may be associated with some embodiments of the perforations;

Figure 11 is a bottom view of another example of a manifold that may be associated with any example embodiments of the dressing or the tissue interface, illustrating additional examples of a pattern that may be associated with some embodiments of the perforations;

Figure 12 is a bottom view of another example of a manifold that may be associated with any example embodiments of the dressing or the tissue interface, illustrating additional embodiments associated with certain illustrative embodiments of the perforations;

Figure 13 is a bottom view of another example of a manifold that may be associated with any example embodiments of the dressing or the tissue interface, illustrating additional example embodiments associated with certain illustrative embodiments of the perforations;

Figure 14 is a bottom view of another example of a manifold that may be associated with any example embodiments of the dressing or the tissue interface, illustrating additional embodiments associated with certain embodiments of the perforations;

Figure 15 is a bottom view of another example of a manifold that may be associated with any example embodiments of the dressing or the tissue interface, illustrating example embodiments that may be associated with some embodiments of the perforations;

Figure 16 is a bottom view of another example of a manifold that may be associated with any example embodiments of the dressing or the tissue interface, illustrating example embodiments where the perforations comprise curved slits or slots;

Figure 17 is a bottom view of another example of a manifold that may be associated with any example embodiments of the dressing or the tissue interface, illustrating some embodiments where the perforations comprise chevron slits or slots;

Figure 18 is a bottom view of another example of a manifold that may be associated with any example embodiments of the dressing or the tissue interface, illustrating example embodiments where the perforations comprise split-chevron slits or slots;

Figure 19 is a schematic view of an example configuration of apertures that may be associated with some embodiments of an optional sealing layer;

Figure 20 is a bottom view showing an example of a manifold that may be associated with any example embodiments of the dressing or the tissue interface with the apertures in the optional sealing layer of Figure 19 overlaid on the manifold;

Figure 21 is a cross-sectional view of the example dressing of Figure 2E, taken at line 21-21, applied to an example tissue site, and illustrating additional details associated with some examples of the therapy system of Figure 1;

Figure 21A illustrates additional details that may be associated with some examples of the dressing;

Figure 21B is a detail view, taken at reference FIG. 21B in Figure 21, illustrating additional details that may be associated with some example embodiments of the dressing;

Figure 22 is a flowchart of an example method for forming some embodiments of a manifold that may be associated with any examples of the dressing or the tissue interface according to this disclosure; and
Figure 23 is an isometric view of an example of a manifold positioned within a mold during formation of a film layer and a non-felted portion, according to some illustrative embodiments.

## DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

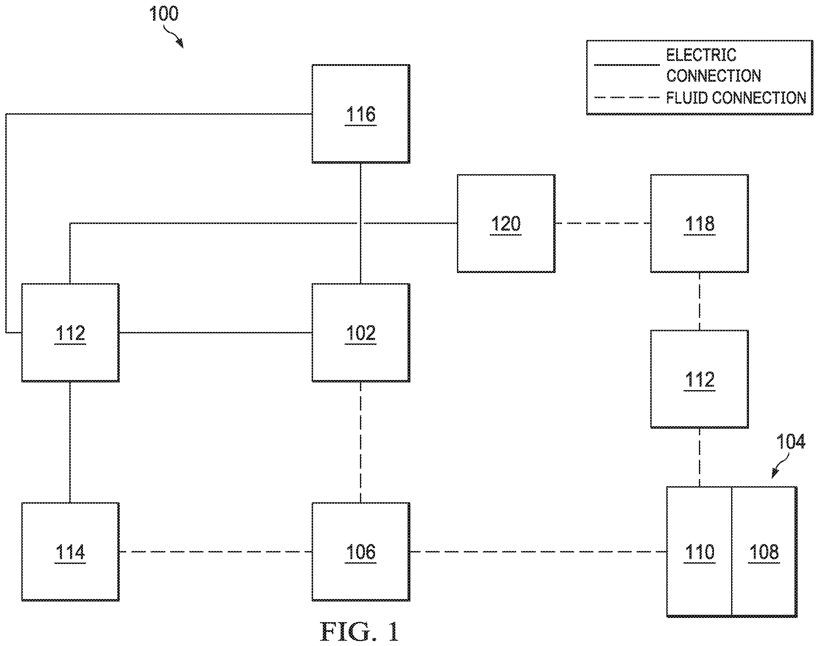

Figure 1 is a block diagram of an example embodiment of a therapy system 100 including an example dressing 104 that can provide negative-pressure therapy and instillation treatment in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 102, and one or more distribution components. A distribution component is preferably detachable and may be disposable, reusable, or recyclable. A dressing, such as a dressing 104, and a fluid container, such as a container 106, are examples of distribution components that may be associated with some examples of the therapy system 100. As illustrated in the example of Figure 1, the dressing 104 may include a tissue interface 108, a cover 110, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include interfaces or fluid ports to facilitate coupling and de-coupling other components. In some embodiments, for example, a dressing interface may facilitate coupling a fluid conductor to the dressing 104. For example, such a dressing interface may be a SENSAT.R.A.C.™ Pad available from Kinetic Concepts, Inc. of San Antonio, Texas.

The therapy system 100 may also include a regulator or controller, such as a controller 112. Additionally, the therapy system 100 may include sensors to measure operating parameters and provide feedback signals to the controller 112 indicative of the operating parameters. As illustrated in Figure 1, for example, the therapy system 100 may include a first sensor 114 and a second sensor 116 coupled to the controller 112.

The therapy system 100 may also include a source of instillation solution. For example, a solution source 118 may be fluidly coupled to the dressing 104, as illustrated in the example embodiment of Figure 1. The solution source 118 may be fluidly coupled to a positive-pressure source such as a positive-pressure source 120, a negative-pressure source such as the negative-pressure source 102, or both in some embodiments. A regulator, such as an instillation regulator 122, may also be fluidly coupled to the solution source 118 and the dressing 104 to ensure proper dosage of instillation solution (e.g. saline) to a tissue site. For example, the instillation regulator 122 may include a piston that can be pneumatically actuated by the negative-pressure source 102 to draw instillation solution from the solution source during a negative-pressure interval and to instill the solution to a dressing during a venting interval. Additionally or alternatively, the controller 112 may be coupled to the negative-pressure source 102, the positive-pressure source 120, or both, to control dosage of instillation solution to a tissue site. In some embodiments, the instillation regulator 122 may also be fluidly coupled to the negative-pressure source 102 through the dressing 104, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the controller 112, the solution source 118, and other components into a therapy unit.

In general, components of the therapy system 100 may be coupled directly or indirectly. For example, the negative-pressure source 102 may be directly coupled to the container 106 and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 112 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 102, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 102 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 106 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 112, may be a microprocessor or computer programmed to operate one or more components of the therapy system 100, such as the negative-pressure source 102. In some embodiments, for example, the controller 112 may be a microcontroller, which generally includes an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the tissue interface 108, for example. The controller 112 is also preferably configured to receive one or more input signals, such as a feedback signal, and programmed to modify one or more operating parameters based on the input signals.

Sensors, such as the first sensor 114 and the second sensor 116, are generally known in the art as any apparatus operable to detect or measure a physical phenomenon or property, and generally provide a signal indicative of the phenomenon or property that is detected or measured. For example, the first sensor 114 and the second sensor 116 may be configured to measure one or more operating parameters of the therapy system 100. In some embodiments, the first sensor 114 may be a transducer configured to measure pressure in a pneumatic pathway and convert the measurement to a signal indicative of the pressure measured. In some embodiments, for example, the first sensor 114 may be a piezo-resistive strain gauge. The second sensor 116 may optionally measure operating parameters of the negative-pressure source 102, such as a voltage or current, in some embodiments. Preferably, the signals from the first sensor 114 and the second sensor 116 are suitable as an input signal to the controller 112, but some signal conditioning may be appropriate in some embodiments. For example, the signal may need to be filtered or amplified before it can be processed by the controller 112. Typically, the signal is an electrical signal, but may be represented in other forms, such as an optical signal.

The tissue interface 108 can be generally adapted to partially or fully contact a tissue site. The tissue interface 108 may take many forms, and may have many sizes, shapes, or thicknesses, depending on a variety of factors, such as the type of treatment being implemented or the nature and size of a tissue site. For example, the size and shape of the tissue interface 108 may be adapted to the contours of deep and irregular shaped tissue sites. Any or all of the surfaces of the tissue interface 108 may have an uneven, coarse, or jagged profile.

In some embodiments, the tissue interface 108 may include or may be a manifold as further described herein. A manifold in this context may include a means for communicating fluid relative to a tissue site under pressure, such as a means for collecting fluid from or distributing fluid to the tissue site. For example, a manifold may be adapted to receive negative pressure from a source and distribute negative pressure through multiple apertures across the tissue interface 108, which may have the effect of collecting fluid from a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may include a plurality of pathways, which can be interconnected to improve distribution or collection of fluids. In some illustrative embodiments, a manifold may include a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively include projections that form interconnected fluid pathways. For example, a manifold may be molded to provide surface projections that define interconnected fluid pathways.

In some embodiments, the tissue interface 108 may include reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 108 may also vary according to needs of a prescribed therapy. For example, the tensile strength of foam may be increased for instillation of topical treatment solutions. The 25% compression load deflection of the tissue interface 108 may be at least 0.35 pounds per square inch, and the 65% compression load deflection may be at least 0.43 pounds per square inch. In some embodiments, the tensile strength of the tissue interface 108 may be at least 10 pounds per square inch. The tissue interface 108 may have a tear strength of at least 2.5 pounds per inch. In some embodiments, the tissue interface may be foam including of polyols such as polyester or polyether, isocyanate such as toluene diisocyanate, and polymerization modifiers such as amines and tin compounds. In some examples, the tissue interface 108 may be reticulated polyurethane foam such as found in GRANUFOAM™ dressing or V.A.C. VERAFLO™ dressing, both available from Kinetic Concepts, Inc. of San Antonio, Texas.

The thickness of the tissue interface 108 may also vary according to needs of a prescribed therapy. For example, the thickness of the tissue interface may be decreased to reduce tension on peripheral tissue. The thickness of the tissue interface 108 can also affect the conformability of the tissue interface 108. In some embodiments, a thickness in a range of about 5 millimeters to 10 millimeters may be suitable.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic material that may be suitable is a polyvinyl alcohol, open-cell foam such as V.A.C. WHITEFOAM™ dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 110 may provide a bacterial barrier and protection from physical trauma. The cover 110 may also be constructed from a material that can reduce evaporative losses and provide a fluid seal between two components or two environments, such as between a therapeutic environment and a local external environment. The cover 110 may include, for example, an elastomeric film or membrane that can provide a seal adequate to maintain a negative pressure at a tissue site for a given negative-pressure source. The cover 110 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 110 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 110 may include, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm® drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inspire 2327 polyurethane films, commercially available from Expopack Advanced Coatings, Wrexham, United Kingdom. In some embodiments, the cover 110 may include INSPIRE 2301 having an MVTR (upright cup technique) of 2600 g/m$^2$/24 hours and a thickness of about 30 microns.

An attachment device may be used to attach the cover 110 to an attachment surface, such as undamaged epidermis, a gasket, or another cover. The attachment device may take many forms. For example, an attachment device may be a medically-acceptable, pressure-sensitive adhesive configured to bond the cover 110 to epidermis around a tissue site. In some embodiments, for example, some or all of the cover 110 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 118 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 108 may be placed within, over, on, or otherwise proximate to a tissue site. If the tissue site is a wound, for example, the tissue interface 108 may partially or completely fill the wound, or it may be placed over the wound. The cover 110 may be placed over the tissue interface 108 and sealed to an attachment surface near a tissue site. For example, the cover 110 may be sealed to undamaged epidermis peripheral to a tissue site. Thus, the dressing 104 can provide a sealed therapeutic environment proximate to a tissue site, substantially isolated from the external environment, and the negative-pressure source 102 can reduce pressure in the sealed therapeutic environment.

In general, exudate and other fluid flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed, without limitation, for purposes of describing various features and components herein. However, the fluid path may also be reversed in some applications, such as by substituting a positive-pressure source for a negative-pressure source.

Negative pressure applied to the tissue site through the tissue interface 108 in the sealed therapeutic environment can induce macro-strain and micro-strain in the tissue site. Negative pressure can also remove exudate and other fluid from a tissue site, which can be collected in container 106.

In some embodiments, the controller 112 may receive and process data from one or more sensors, such as the first sensor 114. The controller 112 may also control the operation of one or more components of the therapy system 100 to manage the pressure delivered to the tissue interface 108. In some embodiments, controller 112 may include an input for receiving a desired target pressure and may be programmed for processing data relating to the setting and inputting of the target pressure to be applied to the tissue interface 108. In some example embodiments, the target pressure may be a fixed pressure value set by an operator as the target negative pressure desired for therapy at a tissue site and then provided as input to the controller 112. The target pressure may vary from tissue site to tissue site based on the type of tissue forming a tissue site, the type of injury or wound (if any), the medical condition of the patient, and the preference of the attending physician. After selecting a desired target pressure, the controller 112 can operate the negative-pressure source 102 in one or more control modes based on the target pressure and may receive feedback from one or more sensors to maintain the target pressure at the tissue interface 108.

In some embodiments, the controller 112 may have a continuous pressure mode, in which the negative-pressure source 102 is operated to provide a constant target negative pressure for the duration of treatment or until manually deactivated. Additionally or alternatively, the controller may have an intermittent pressure mode. In example embodiments, the controller 112 can operate the negative-pressure source 102 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 102, which can form a square wave pattern between the target pressure and atmospheric pressure.

In some example embodiments, the increase in negative-pressure from ambient pressure to the target pressure may not be instantaneous. For example, the negative-pressure source 102 and the dressing 104 may have an initial rise time. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min. and a descent rate set at about 30 mmHg/min.

In some embodiments, the controller 112 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 112, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 112 may receive and process data, such as data related to installation solution provided to the tissue interface 108. Such data may include the type of installation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 112 may also control the operation of one or more components of the therapy system 100 to instill solution. For example, the controller 112 may manage fluid distributed from the solution source 118 to the tissue interface 108. In some embodiments, fluid may be instilled to a tissue site by applying a negative pressure from the negative-pressure source 102 to reduce the pressure at the tissue site, drawing solution into the tissue interface 108. In some embodiments, solution may be instilled to a tissue site by applying a positive pressure from the positive-pressure source 120 to move solution from the solution source 118 to the tissue interface 108. Additionally or alternatively, the solution source 118 may be elevated to a height sufficient to allow gravity to move solution into the tissue interface 108.

The controller 112 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 108, or it may be implemented to provide a dynamic pressure mode of operation to vary the flow rate of instillation solution through the tissue interface 108. Alternatively, the application of negative pressure may be implemented to provide an intermittent mode of operation to allow instillation solution to dwell at the tissue interface 108. In an intermittent mode, a specific fill volume and dwell time may be provided depending, for example, on the type of tissue site being treated and the type of dressing being utilized. After or during instillation of solution, negative-pressure treatment may be applied. The controller 112 may be utilized to select a mode of operation and the duration of the negative pressure treatment before commencing another instillation cycle by instilling more solution.

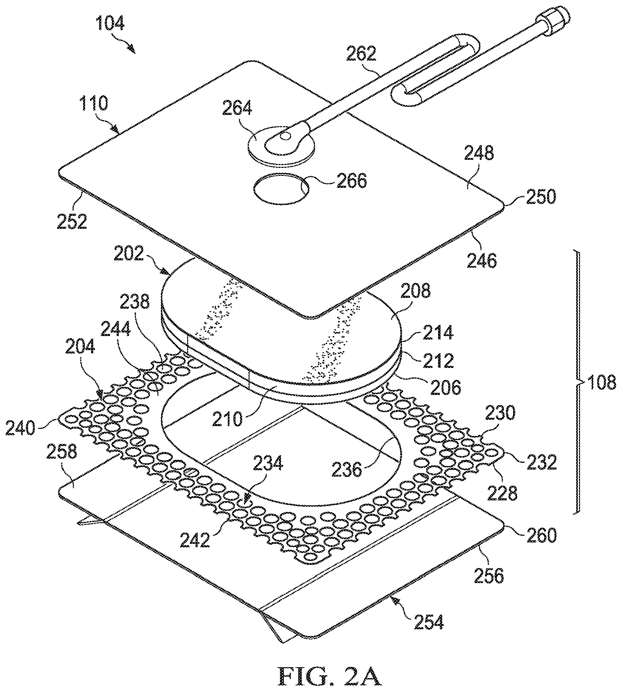

Figure 2A is an exploded view of an example of the dressing 104 of Figure 1, illustrating additional details that may be associated with some embodiments in which a tissue interface 108 that may be associated with the dressing 104 includes more than one layer. In the example of Figure 2A, the tissue interface 108 may include a first layer, such as a manifold 202, and a second layer, such as a sealing layer 204. The dressing 104 may also include the cover 110.

In some embodiments, the manifold 202 may be formed from a porous foam, including the previously described foams of the GRANUFOAM™ dressing, the reticulated polyurethane foam of the V.A.C. VERAFLO™ dressing, or the polyvinyl alcohol, open-celled V.A.C. WHITEFOAM™. For example, a portion of the manifold 202 may be formed by a felting process. Felting may be performed by any known methods, which may include applying heat and pressure to a porous material or foam material. Some methods may include compressing a foam blank between one or more heated platens or dies (not shown) for a specified period of time and at a specified temperature. The direction of compression may be along the thickness of the foam blank. The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material.

The felting process may alter certain properties of the original material, including pore shape and/or size, elasticity, density, and density distribution. For example, struts that define pores in the foam may be deformed during the felting process, resulting in flattened pore shapes. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface. In some embodiments, the felted structure may be comparatively smoother than any unfinished or non-felted surface or portion of the porous material. Further, the pores in the felted structure may be smaller than the pores throughout any unfinished or non-felted surface or portion of the porous material.

A felted foam may be characterized by a firmness factor, which is indicative of the compression of the foam. The firmness factor of a felted foam can be specified as the ratio of original thickness to final thickness. A compressed or felted foam may have a firmness factor greater than 1. The degree of compression may affect the physical properties of the felted foam. For example, felted foam has an increased effective density compared to a foam of the same material that is not felted. The felting process can also affect fluid-to-foam interactions. For example, as the density increases, compressibility or collapse may decrease. Therefore, foams which have different compressibility or collapse may have different firmness factors. In some example embodiments, a firmness factor can range from about 2 to about 10, preferably about 3 to about 7. There is a general linear relationship between firmness level, density, pore size (or pores per inch) and compressibility. The physical properties of a felted foam in relation to the physical properties of a pre-felted or unfelted foam from which the felted foam is formed may be determined by one or more of the following equations, such as Equations 1-6 below:

$$Density_{Felted} = Density_{Unfelted} \times Firmness\ Factor; \qquad (1)$$

$$Average\ Pore\ Size_{Felted} = \frac{Average\ Pore\ Size_{Unfelted}}{Firmness\ Factor}; \qquad (2)$$

$$Free\ Volume_{Felted} = \frac{Free\ Volume_{Unfelted}}{Firmness\ Factor}; \qquad (3)$$

$$Average\ Porosity_{Felted} = Average\ Porosity_{Unfelted} \times Firmness\ Factor; \qquad (4)$$

$$25\%\ Compression\ Load\ Deflection_{Felted} = \qquad (5)$$
$$25\%\ Compression\ Load\ Deflection_{Unfelted} \times Firmness\ Factor; \text{and}$$

$$65\%\ Compression\ Load\ Deflection_{Felted} = \qquad (6)$$
$$65\%\ Compression\ Load\ Deflection_{Unfelted} \times Firmness\ Factor.$$

For example, foam that is felted to a firmness factor of 3 will show a three-fold density increase and compress to about a third of its original thickness. Foam that is felted to a firmness factor of 3 may show a three-fold decrease in pore size, a three-fold decrease in free volume, a three-fold increase in average pores per inch, a three-fold increase in 25% compression load deflection, and a three-fold increase in 65% compression load deflection.

In some embodiments, the manifold 202 may include a tissue-facing surface or a tissue contact surface such as a first surface 206 opposite a second surface 208, and a periphery 210. In some embodiments, a portion of the manifold 202 may be felted, such as a felted portion 212. In some embodiments, the felted portion 212 may extend a depth away from the first surface 206 and into the manifold 202. In some embodiments, the first surface 206 may be a felted surface. In some embodiments, the remainder of the manifold 202 may be non-felted, such as non-felted portion 214. In some embodiments, non-felted portion may extend a depth away from the second surface 208 and into the manifold 202. In some embodiments, the non-felted portion 214 may have the characteristics of the pre-felted foam blank used to form the manifold 202. For example, a suitable foam blank (e.g., of pre-felted foam) may be a foam such as GRANUFOAM™, and may have a density in a range of about 1.3 lb/ft$^3$ to about 1.6 lb/ft$^3$, an average pore size in a range of about 400 micrometers to about 600 micrometers, a free volume of about 90%, an average porosity in a range of about 40 pores per inch to about 50 pores per inch, a 25% compression load deflection of about 0.35 psi, and a 65% compression load deflection of about 0.43 psi. In some embodiments, a portion of the manifold 202, such as the felted portion 212, may be felted to provide a denser foam for the manifold 202. In some embodiments, the felted portion 212 may be felted to a firmness factor in a range of between about 2 and 10. The felted portion 212 may have a density in a range of about 2.6 lb/ft$^3$ to about 14.4 lb/ft$^3$, an average pore size in a range of about 40 micrometers to about 300 micrometers, a free volume in a range of about 9% to about 45%, an average porosity in a range of about 80 pores per inch to about 500 pores per inch, a 25% compression load deflection in a range of about 0.7 psi to about 3.5 psi, and a 65% compression load deflection in a range of about 0.86 psi to about 4.3 psi.

In some embodiments, the felted portion 212 may be felted to a firmness factor of about 2. The felted portion 212 may have a density in a range of about 2.6 lb/ft$^3$ to about 3.2 lb/ft$^3$, an average pore size in a range of about 200 micrometers to about 300 micrometers, a free volume of about 45%, an average porosity in a range of about 80 pores per inch to about 100 pores per inch, a 25% compression load deflection of about 0.7 psi, and a 65% compression load deflection of about 0.86 psi.

In some embodiments, the felted portion 212 may be felted to a firmness factor of about 3. The felted portion 212 may have a density in a range of about 3.9 lb/ft$^3$ to about 4.8 lb/ft$^3$, an average pore size in a range of about 133.33 micrometers to about 200 micrometers, a free volume of about 30%, an average porosity in a range of about 120 pores per inch to about 150 pores per inch, a 25% compression load deflection of about 1.05 psi, and a 65% compression load deflection of about 1.29 psi.

In some embodiments, the felted portion 212 may be felted to a firmness factor of about 4. The felted portion 212 may have a density in a range of about 5.2 lb/ft$^3$ to about 6.4 lb/ft$^3$, an average pore size in a range of about 100 micrometers to about 150 micrometers, a free volume of about 23%, an average porosity in a range of about 160 pores per inch to about 200 pores per inch, a 25% compression load deflection of about 1.4 psi, and a 65% compression load deflection of about 1.72 psi.

In some embodiments, the felted portion 212 may be felted to a firmness factor of about 5. The felted portion 212 may have a density in a range of about 6.5 lb/ft$^3$ to about 8 lb/ft$^3$, an average pore size in a range of about 80 micrometers to about 120 micrometers, a free volume of about 18%, an average porosity in a range of about 200 pores per inch to about 250 pores per inch, a 25% compression load deflection of about 1.75 psi, and a 65% compression load deflection of about 2.15 psi.

In some embodiments, the felted portion 212 may be felted to a firmness factor of about 6. The felted portion 212 may have a density in a range of about 7.8 lb/ft$^3$ to about 9.6 lb/ft$^3$, an average pore size in a range of about 66.67 micrometers to about 100 micrometers, a free volume of about 15%, an average porosity in a range of about 240 pores per inch to about 300 pores per inch, a 25% compression load deflection of about 2.1 psi, and a 65% compression load deflection of about 2.58 psi.

In some embodiments, the felted portion 212 may be felted to a firmness factor of about 7. The felted portion 212 may have a density in a range of about 9.1 lb/ft$^3$ to about 11.2 lb/ft$^3$, an average pore size in a range of about 57.14 micrometers to about 85.71 micrometers, a free volume of about 13%, an average porosity in a range of about 280 pores per inch to about 350 pores per inch, a 25% compression load deflection of about 2.45 psi, and a 65% compression load deflection of about 3.01 psi.

In some embodiments, the felted portion 212 may be felted to a firmness factor of about 8. The felted portion 212 may have a density in a range of about 10.4 lb/ft$^3$ to about 12.8 lb/ft$^3$, an average pore size in a range of about 50 micrometers to about 75 micrometers, a free volume of about 11%, an average porosity in a range of about 320 pores per inch to about 400 pores per inch, a 25% compression load deflection of about 2.8 psi, and a 65% compression load deflection of about 3.44 psi.

In some embodiments, the felted portion 212 may be felted to a firmness factor of about 9. The felted portion 212 may have a density in a range of about 11.7 lb/ft$^3$ to about 14.4 lb/ft$^3$, an average pore size in a range of about 44.44 micrometers to about 66.67 micrometers, a free volume of about 10%, an average porosity in a range of about 360 pores per inch to about 450 pores per inch, a 25% compression load deflection of about 3.15 psi, and a 65% compression load deflection of about 3.87 psi.

In some embodiments, the felted portion 212 may be felted to a firmness factor of about 10. The felted portion 212 may have a density in a range of about 13 lb/ft$^3$ to about 16 lb/ft$^3$, an average pore size in a range of about 40 micrometers to about 60 micrometers, a free volume of about 9%, an average porosity in a range of about 400 pores per inch to about 500 pores per inch, a 25% compression load deflection of about 3.5 psi, and a 65% compression load deflection of about 4.3 psi.

According to illustrative embodiments, the pre-felted foam blank may be a foam such as foams found in V.A.C. VERAFLOW™ dressings, and may have a density in a range of about 1.7 lb/ft$^3$ to about 2.1 lb/ft$^3$, an average pore size in a range of about 400 micrometers to about 600 micrometers, an average porosity in a range of about 40 pores per inch to about 50 pores per inch, a 25% compression load deflection of about 0.35 psi, and a 65% compression load deflection of about 0.6 psi. In some embodiments, a portion of the manifold 202, such as the felted portion 212 may be felted to provide a denser foam for the manifold. In some embodiments, the felted portion 212 may be felted to a firmness factor in a range of between about 2 and 10. The felted portion 212 may have a density in a range of about 3.4 lb/ft$^3$ to about 21 lb/ft$^3$, an average pore size in a range of about 40 micrometers to about 300 micrometers, an average porosity in a range of about 80 pores per inch to about 500 pores per inch, a 25% compression load deflection in a range of about 0.7 psi to about 3.5 psi, and a 65% compression load deflection in a range of about 0.86 psi to about 4.3 psi.

In some embodiments, the felted portion 212 may be felted to a firmness factor of about 2. The felted portion 212 may have a density in a range of about 3.4 lb/ft$^3$ to about 4.2 lb/ft$^3$, an average pore size in a range of about 200 micrometers to about 300 micrometers, an average porosity in a range of about 80 pores per inch to about 100 pores per inch, a 25% compression load deflection of about 0.7 psi, and a 65% compression load deflection of about 0.6 psi.

In some embodiments, the felted portion 212 may be felted to a firmness factor of about 3. The felted portion 212 may have a density in a range of about 5.1 lb/ft$^3$ to about 6.3 lb/ft$^3$, an average pore size in a range of about 133.33 micrometers to about 200 micrometers, an average porosity in a range of about 120 pores per inch to about 150 pores per inch, a 25% compression load deflection of about 1.05 psi, and a 65% compression load deflection of about 1.8 psi.

In some embodiments, the felted portion 212 may be felted to a firmness factor of about 4. The felted portion 212 may have a density in a range of about 6.8 lb/ft$^3$ to about 8.4 lb/ft$^3$, an average pore size in a range of about 100 micrometers to about 150 micrometers, an average porosity in a range of about 160 pores per inch to about 200 pores per inch, a 25% compression load deflection of about 1.4 psi, and a 65% compression load deflection of about 2.4 psi.

In some embodiments, the felted portion 212 may be felted to a firmness factor of about 5. The felted portion 212 may have a density in a range of about 8.5 lb/ft$^3$ to about 10.5 lb/ft$^3$, an average pore size in a range of about 80 micrometers to about 120 micrometers, an average porosity in a range of about 200 pores per inch to about 250 pores per inch, a 25% compression load deflection of about 1.75 psi, and a 65% compression load deflection of about 3 psi.

In some embodiments, the felted portion 212 may be felted to a firmness factor of about 6. The felted portion 212 may have a density in a range of about 10.2 lb/ft$^3$ to about 12.6 lb/ft$^3$, an average pore size in a range of about 66.67 micrometers to about 100 micrometers, an average porosity in a range of about 240 pores per inch to about 300 pores per inch, a 25% compression load deflection of about 2.1 psi, and a 65% compression load deflection of about 3.6 psi.

In some embodiments, the felted portion 212 may be felted to a firmness factor of about 7. The felted portion 212 may have a density in a range of about 11.9 lb/ft$^3$ to about 14.7 lb/ft$^3$, an average pore size in a range of about 57.14 micrometers to about 85.71 micrometers, an average porosity in a range of about 280 pores per inch to about 350 pores per inch, a 25% compression load deflection of about 2.45 psi, and a 65% compression load deflection of about 4.2 psi.

In some embodiments, the felted portion 212 may be felted to a firmness factor of about 8. The felted portion 212 may have a density in a range of about 13.6 lb/ft$^3$ to about 16.8 lb/ft$^3$, an average pore size in a range of about 50 micrometers to about 75 micrometers, an average porosity in a range of about 320 pores per inch to about 400 pores per inch, a 25% compression load deflection of about 2.8 psi, and a 65% compression load deflection of about 4.8 psi.

In some embodiments, the felted portion 212 may be felted to a firmness factor of about 9. The felted portion 212 may have a density in a range of about 15.3 lb/ft$^3$ to about 18.9 lb/ft$^3$, an average pore size in a range of about 44.44 micrometers to about 66.67 micrometers, an average porosity in a range of about 360 pores per inch to about 450 pores per inch, a 25% compression load deflection of about 3.15 psi, and a 65% compression load deflection of about 5.4 psi.

In some embodiments, the felted portion 212 may be felted to a firmness factor of about 10. The felted portion 212 may have a density in a range of about 17 lb/ft$^3$ to about 21 lb/ft$^3$, an average pore size in a range of about 40 micrometers to about 60 micrometers, an average porosity in a range of about 400 pores per inch to about 500 pores per inch, a 25% compression load deflection of about 3.5 psi, and a 65% compression load deflection of about 6 psi.

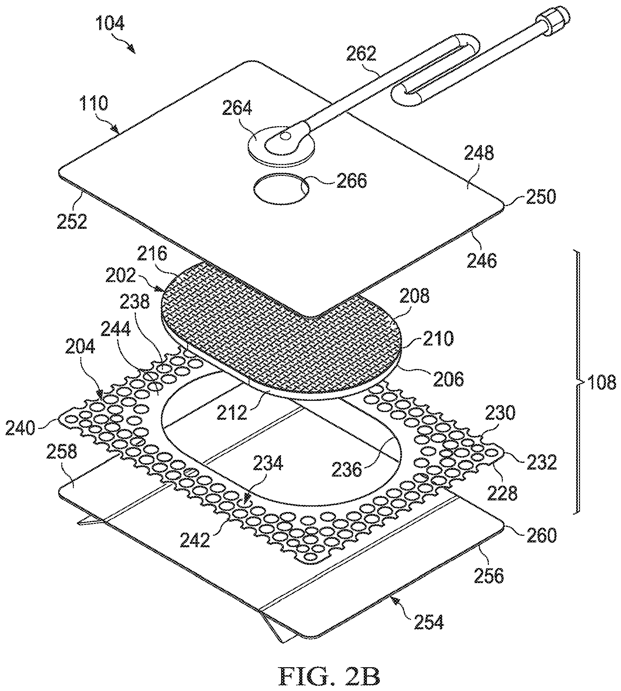

Figure 2B is an exploded view of an example of the dressing 104 of Figure 1, illustrating additional details that may be associated with some embodiments in which a tissue interface 108 that may be associated with the dressing 104 includes more than one layer. In the example of Figure 2B, the manifold 202 may be felted throughout its thickness such that the felted portion 214 extends from the first surface 206 though the manifold 202 to the second surface 208. In some embodiments, both the first surface 206 and the second surface 208 may be felted surfaces. As shown in Figure 2B, in some embodiments, the manifold 202 may not have a non-felted portion 214.

In some embodiments, one or more perforations 216 may be formed through the felted portion 212. In examples where the felted portion 212 extends a distance into the manifold 202 from the first surface 206 but not through entire thickness of the manifold 202, for example, as illustrated in Figure 2A, the perforations 216 may extend from the first surface 206 to non-felted portion 214, providing fluid communication between the first surface 206 and the non-felted portion 214, through the thickness of the felted portion 212. As illustrated in Figure 2B, in examples of the manifold 202 where the manifold may be felted throughout its thickness, the perforations 216 may extend through the entire thickness of the felted portion 214 and the manifold 202, from the first surface 206 to the second surface 208. The perforations 216 may provide fluid communication between the first surface 206 and the second surface 208 through the thickness of the felted portion 212 and the manifold 202. The perforations 216 may be formed by removing material from the felted portion 212. For example, perforations 216 may be formed by cutting through the felted portion 212. In some embodiments, each of the perforations 216 may be a slot having a length in a range of about 1 millimeter to about 5 millimeters, and a width in a range of about 0.4 millimeters to about 1 millimeter.

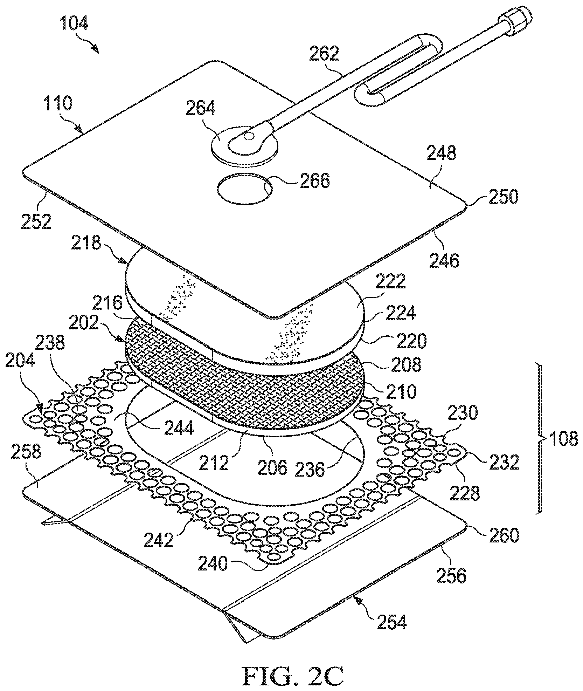

Figure 2C is an exploded view of an example of the dressing 104 of Figure 1, illustrating additional details that may be associated with some embodiments in which a tissue interface 108 that may be associated with the dressing 104 includes more than one layer. In the example of Figure 2C, the dressing 104 includes a secondary manifold 218. The secondary manifold 218 may comprise a porous foam, such as any foam previously described with respect to the manifold 202. The secondary manifold 218 may be formed from a non-felted foam. In some embodiments, the secondary manifold 218 may include a tissue-facing surface, such as a first surface 220 opposite a second surface 222, and a periphery 224. In some embodiments, the periphery 224 of the secondary manifold 218 may be substantially coextensive with the periphery 210 of the manifold 202.

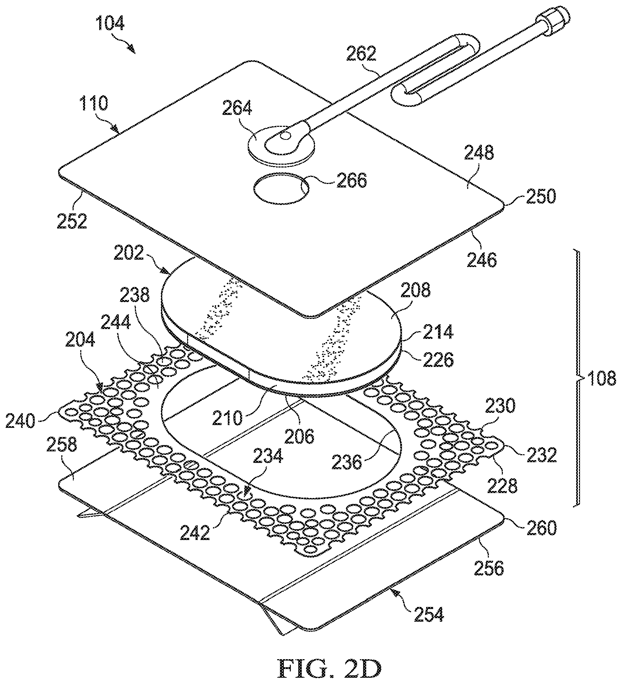

Figure 2D is an exploded view of an example of the dressing 104 of Figure 1, illustrating additional details that may be associated with some embodiments in which a tissue interface 108 that may be associated with the dressing 104 includes more than one layer. In the example of Figure 2D, the manifold 202 may not be felted. In some embodiments, the manifold 202 may include a non-felted portion 214, but not a felted portion 212. In some embodiments, the manifold 202 may include an integrally formed film layer 226 on the first surface 206. For example, the film layer 226 may include polymeric film that is integral to the overall structure of the manifold 202. In some embodiments, the film layer 226 may include a hydrophobic polymeric film. In illustrative embodiments, the film layer 226 may be a polyurethane film formed on the first surface 206 of the manifold 202 during manufacture of the manifold 202. In some embodiments, the film layer 226 may include a polyurethane film that is integral to the polyurethane foam of the manifold 202. For example, the film layer 226 may be formed from the same material as the manifold 202 such that the manifold 202 integrally transitions from a foam portion, such as the non-felted portion 214 to the film layer 226. In some embodiments, the structure of the non-felted portion 214 may transition to the film layer 226. In some embodiments, the film layer 226 may be attached to the non-felted portion 214 without the use of an adhesive or other coupling means. Other suitable polymeric materials for forming the non-felted portion 214 and the film layer 226 may include silicones, elastomeric polyesters (for example, HYTREL® elastomers), polyether copolymers (such as PEBAX® elastomers), and isocyanate-free polyurethanes (for example, amineoplast/carbamate copolymers, polycarbamate/polyamine materials, or polycarbamate/polyaldehyde materials).

The film layer 226 may have material properties that make it conducive to being applied against a tissue site. For example, the film layer 226 may have a thickness of between 20 micrometers and 100 micrometers. In some embodiments, the hydrophobicity of the film layer 226 may be modified or enhanced with a hydrophobic coating of other materials, such as silicones and fluorocarbons, either as coated from a liquid or plasma coated. In some embodiments, the perforations 216 may be formed through the thickness of the film layer 226 to provide the porous space of the non-felted portion 214 with fluid communication through the film layer 226. In some embodiments, the perforations 216 may provide fluid communication between the porous spaces of the non-felted portion 214 with an environment opposite the film layer 226 from the non-felted portion 214. In some embodiments, the film layer 226 may serve as a tissue in-growth barrier, substantially preventing granulation tissue from growing into the manifold 202.

As illustrated in the examples of Figures 2A-2D, the dressing 104 may also include a sealing layer 204 formed from a soft, pliable material suitable for providing a fluid seal with a tissue site, such as a suitable gel material. In some embodiments, the sealing layer 204 may include a silicone gel, a soft silicone, a hydrocolloid, a hydrogel, a polyurethane gel, a polyolefin gel, a hydrogenated styrenic copolymer gel, a foamed gel, a soft closed cell foam (such as polyurethanes and polyolefins coated with an adhesive), polyurethane, polyolefin, or hydrogenated styrenic copolymers. In some embodiments, the sealing layer 204 may include a tissue-facing surface, such as a first surface 228 opposite a second surface 230, and a periphery 232. In some embodiments, the first surface 228 and the second surface 230 may be substantially flat surfaces. In some embodiments, the sealing layer 204 may have a thickness between the first surface 228 and the second surface 230 in a range of about 200 micrometers to about 1,000 micrometers.

As shown in the examples of Figures 2A-2D, some embodiments of the sealing layer 204 may have an outer region 234 between the periphery 232 and a treatment aperture 236 formed through the sealing layer 204. In some embodiments, a plurality of apertures 238 may be formed through the sealing layer 204 in the periphery 232 surrounding the treatment aperture 236. The treatment aperture 236 may be complementary or correspond to a surface area of the manifold 202 bound by the periphery 210. For example, the treatment aperture may form a frame, window, or other opening around a surface of the manifold 202, such as a region of the first surface 206 near the periphery 210. The sealing layer 204 may also include corners 240 and edges 242. The corners 240 and edges 242 may be a part of the periphery 232. The sealing layer 204 may also include an interior border 244 around the treatment aperture 236, which may be substantially free of the apertures 238. In some examples, as illustrated in Figures 2A-2D, the treatment aperture 236 may be symmetrical and centrally disposed in the sealing layer 204, forming an open central window.

The apertures 238 may be formed by cutting, perforating, or by application of local RF or ultrasonic energy, for example, or by other suitable techniques for forming an opening or perforation in the sealing layer 204. The apertures 238 may have a uniform distribution pattern, or may be randomly distributed on the sealing layer 204. The apertures 238 in the sealing layer 204 may have many shapes, including circles, squares, stars, ovals, polygons, slits, complex curves, rectilinear shapes, triangles, for example, or may have some combination of such shapes.

Each of the apertures 238 may have uniform or similar geometric properties. For example, in some embodiments, each of the apertures 238 may be circular apertures, having substantially the same diameter. In some embodiments, each of the apertures 238 may have a diameter of about 1 millimeter to about 50 millimeters. In other embodiments, the diameter of each of the apertures 238 may be about 1 millimeter to about 20 millimeters.

In some embodiments, geometric properties of the apertures 238 may vary. For example, the diameter of the apertures 238 may vary depending on the position of the apertures 238 in the sealing layer 204. For example, in some embodiments, the apertures 238 disposed in the outer region 234 may have a diameter between about 5 millimeters and about 10 millimeters. A range of about 7 millimeters to about 9 millimeters may be suitable for some examples. In some embodiments, the apertures 238 disposed near the corners 240 may have a diameter between about 7 millimeters and about 8 millimeters.

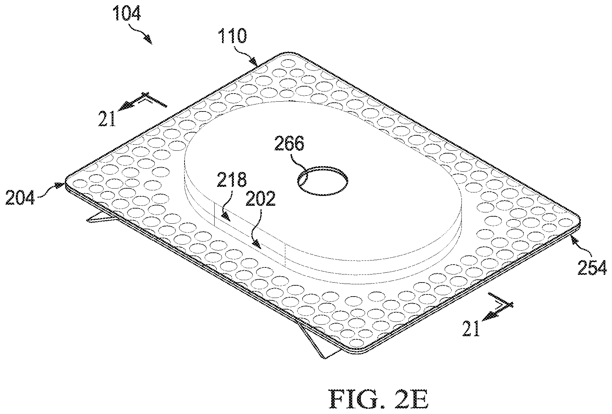

At least one of the apertures 238 in the outer region 234 of the sealing layer 204 may be positioned at the edges 242 of the outer region 234, and may have an interior cut open or exposed at the edges 242 that is in fluid communication in a lateral direction with the edges 242. The lateral direction may refer to a direction toward the edges 242 and in the same plane as the sealing layer 204. As shown in the example of Figure 2, the apertures 238 in the outer region 234 may be positioned proximate to or at the edges 242 and in fluid communication in a lateral direction with the edges 242. The apertures 238 positioned proximate to or at the edges 242 may be spaced substantially equidistant around the outer region 234 as shown in Figures 2A-2D. Alternatively, the spacing of the apertures 238 proximate to or at the edges 242 may be irregular.

As illustrated in the Figures 2A-2D, the cover 110 may include a tissue-facing surface, such as a first surface 246 opposite a second surface 248, and a periphery 250. In some embodiments, the cover 110 may include an attachment device, such as an adhesive 252 disposed on the first surface 246 of the cover 110. The adhesive 252 may be, for example, a medically-acceptable, pressure-sensitive adhesive that extends about a periphery, a portion, or an entire surface of the cover 110. In some embodiments, for example, the adhesive 252 may be an acrylic adhesive having a coating weight between 25-65 grams per square meter (g.s.m.). Thicker adhesives, or combinations of adhesives, may be applied in some embodiments to improve the seal and reduce leaks. In some embodiments, such a layer of the adhesive 252 may be continuous or discontinuous. Discontinuities in the adhesive 252 may be provided by apertures or holes (not shown) in the adhesive 252. The apertures or holes in the adhesive 252 may be formed after application of the adhesive 252 or by coating the adhesive 252 in patterns on a carrier layer, such as, for example, a side of the cover 110. Apertures or holes in the adhesive 252 may also be sized to enhance the MVTR of the dressing 104 in some example embodiments. In some embodiments, the periphery 250 of the cover 110 may be substantially coextensive with the periphery 232 of the sealing layer 204.

In some embodiments, the dressing 104 may include a release liner 254 to protect the adhesive 252 prior to application of the dressing 104. The release liner 254 may also provide stiffness to assist with, for example, the deployment of the dressing 104. The release liner 254 may be, for example, a casting paper, a film, or polyethylene. Further, in some embodiments, the release liner 254 may be a polyester material such as polyethylene terephthalate (PET), or similar polar semi-crystalline polymer. The use of a polar semi-crystalline polymer for the release liner 254 may substantially preclude wrinkling or other deformation of the dressing 104. For example, the polar semi-crystalline polymer may be highly oriented and resistant to softening, swelling, or other deformation that may occur when brought into contact with components of the dressing 104, or when subjected to temperature or environmental variations, or sterilization. The release liner 254 may include a first surface 256 opposite a second surface 258, and a periphery 260. In some embodiments, a release agent may be disposed on a side of the release liner 254 that is configured to contact the sealing layer 204, manifold 202, and/or the cover 110. For example, the release agent may be disposed on the second surface 258 of the release liner 254, and be configured to contact the first surface 228 of the sealing layer 204, the first surface 206 of the manifold 202, and/or the adhesive 252 disposed on the first surface 246 of the cover. In some examples, the release agent may be a silicone coating and may have a release factor suitable to facilitate removal of the release liner 254 by hand and without damaging or deforming the dressing 104. In some embodiments, the release agent may be a fluorocarbon or a fluorosilicone, for example. In some embodiments, the release liner 254 may be uncoated or otherwise used without a release agent.

Figures 2A-2D also illustrate examples of a fluid conductor 262 and a dressing interface 264. As shown in Figures 2A-2D, some examples of the fluid conductor 262 may be a flexible tube, which can be fluidly coupled to the dressing interface 264 on one end of the fluid conductor 262. The dressing interface 264 may be an elbow connector which can be placed over an aperture 266 formed through the cover 110 to provide a fluid path between the fluid conductor 262 and the manifold 202.

One or more of the components of the dressing 104 may additionally be treated with an antimicrobial agent in some embodiments. For example, the manifold 202 and/or the secondary manifold 218 may be coated with an antimicrobial agent. In some embodiments, the manifold 202 and/or the secondary manifold 218 may include antimicrobial elements, such as fibers coated with an antimicrobial agent. Additionally or alternatively, some embodiments of the manifold 202 and/or the secondary manifold 218 may be a polymer coated or mixed with an antimicrobial agent. In example embodiments, the fluid conductor 262 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Additionally or alternatively, one or more of the components may be coated with a mixture that may include citric acid and collagen, which can reduce bio-films and infections. For example, the primary manifold 202 and/or the secondary manifold 218 may be a foam coated with such a mixture.

In some embodiments, the dressing 104 may be assembled in a stacked relationship with the cover 110 and the sealing layer 204 enclosing the manifold 202. For example, as illustrated in Figures 2A, 2B, and 2D, when the dressing 104 is assembled, at least a portion of the first surface 206 of the manifold 202 may be disposed adjacent to a portion of the second surface 230 of the sealing layer 204. For example, a portion of the first surface 206 near the periphery 210 of the manifold 202 may be disposed adjacent to a portion of the second surface 230 near the interior border 244. In some embodiments, a portion of the first surface 206 may be adhered to a portion of the second surface 230. In some embodiments, at least a portion of the first surface 246 of the cover 110 may be disposed adjacent to at least a portion of the second surface 208 of the manifold 202 and a portion of the second surface 230 of the sealing layer 204. For example, at least a portion of the first surface 246 of the cover 110 near the aperture 266 may be adhered to at least a portion of the second surface 208 of the manifold 202 with the adhesive 252, and at least a portion of the first surface 246 of the cover 110 near the periphery 250 may be adhered to at least a portion of the second surface 230 of the sealing layer 204 at the outer region 234 with the adhesive 252. In some embodiments, in assembled form, the periphery 250 of the cover 110 may be substantially coextensive with the periphery 232 of the sealing layer 204, and the periphery 210 of the manifold 202 may be substantially contained within the periphery 232 and the periphery 250.

In some embodiments, the dressing 104 may be assembled in a stacked relationship with the cover 110 and the sealing layer 204 enclosing the manifold 202 and the secondary manifold 218. For example, as illustrated in Figure 2C, the manifold 202 and the secondary manifold 218 may be assembled in a stacked relationship such that at least a portion of the first surface 220 of the secondary manifold 218 may be disposed adjacent to at least a portion of the second surface 208 of the manifold 202. In some embodiments, at least a portion of the first surface 206 of the manifold 202 may be disposed adjacent to a portion of the second surface 230 of the sealing layer 204. For example, a portion of the first surface 206 near the periphery 210 of the manifold 202 may be disposed adjacent to a portion of the second surface 230 near the interior border 244. In some embodiments, a portion of the first surface 206 may be adhered to a portion of the second surface 230. In some embodiments, at least a portion of the first surface 246 of the cover 110 may be disposed adjacent to at least a portion of the second surface 222 of the secondary manifold 218 and a portion of the second surface 230 of the sealing layer 204. For example, at least a portion of the first surface 246 of the cover 110 near the aperture 266 may be adhered to at least a portion of the second surface 222 of the secondary manifold 218 with the adhesive 252, and at least a portion of the first surface 246 of the cover 110 near the periphery 250 may be adhered to at least a portion of the second surface 230 of the sealing layer 204 at the outer region 234 with the adhesive 252. In some embodiments, in assembled form, the periphery 224 of the secondary manifold 218 may be substantially coextensive with the periphery 210 of the manifold 202. In some embodiments, in assembled form, the periphery 250 of the cover 110 may be substantially coextensive with the periphery 232 of the sealing layer 204, and the periphery 224 of the secondary manifold 218 and the periphery 210 of the manifold 202 may be substantially contained within the periphery 232 and the periphery 250.

Figure 2E is an isometric view of an assembled example of the dressing 104 of Figure 2C. As shown in the example of Figure 2E, the cover 110 may be substantially clear or optically transparent, allowing for visualization of the secondary manifold 218, the manifold 202, and the sealing layer 204 through the cover 110.

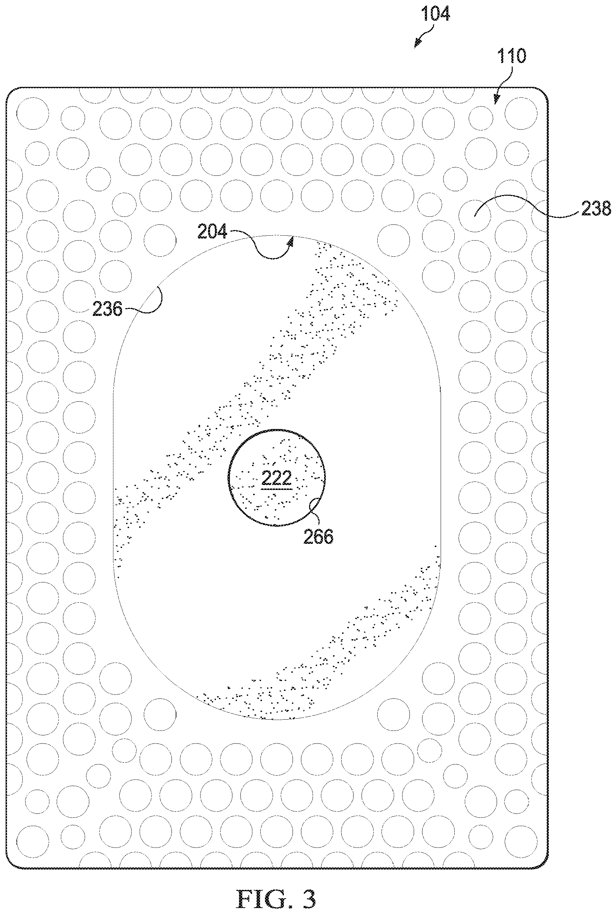

Figure 3 is a top view of the dressing 104 in the example of Figure 2C, as assembled, illustrating additional details that may be associated with some embodiments. As illustrated in the example of Figure 2C, the cover 110 and the sealing layer 204 may have substantially the same perimeter shape and dimensions, so that the cover 110 and the sealing layer 204 are coextensive in some examples. The cover 110 may be substantially transparent, allowing visibility of the apertures 238 in some embodiments. The secondary manifold 218 may be centrally disposed over the manifold 202 (not visible in Figure 3), such as over the treatment aperture 236 (not visible in Figure 3). The cover 110 may be disposed over the secondary manifold 218 and manifold 202 and coupled to the sealing layer 204 around the secondary manifold 218 and manifold 202 so that at least some of the adhesive 252 can be disposed adjacent to the apertures 238.

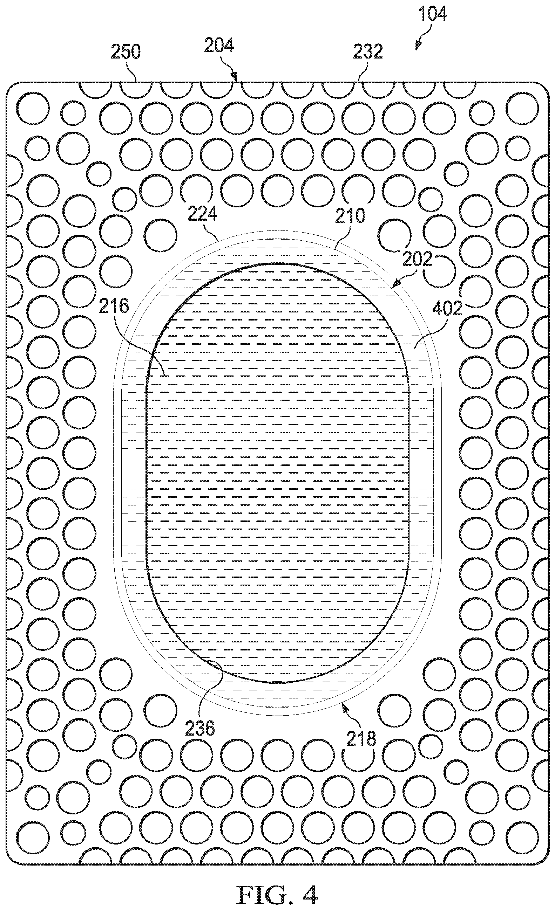

Figure 4 is a bottom view of the dressing 104 in the example of Figure 2C, as assembled, illustrating additional details that may be associated with some embodiments. As illustrated in the example of Figure 4, a substantial number of the perforations 216 may be aligned or otherwise exposed through the treatment aperture 236. In some embodiments, the manifold 202 and the secondary manifold 218 may be substantially aligned with the treatment aperture 236, or may extend across the treatment aperture 236.

Additionally, the secondary manifold 218 may have a periphery 224, and the manifold 202 may have a periphery 210. In some examples, the periphery 224 and the periphery 210 may have substantially the same shape so that adjacent faces of the secondary manifold 218 and the manifold 202 are geometrically similar. The periphery 224 and the periphery 210 may also be congruent in some examples, so that adjacent faces of the secondary manifold 218 and the manifold 202 are substantially coextensive and have substantially the same surface area. In the example of Figure 4, the periphery 224 defines a larger face of the secondary manifold 218 than the face of the manifold 202 defined by the periphery 210, and the larger face of the secondary manifold 218 extends past the smaller face of the periphery 210.

The faces defined by the periphery 210, the periphery 224, or both may also be geometrically similar to the treatment aperture 236 in some embodiments, as illustrated in the example of Figure 4, or may be larger than the treatment aperture 236. The sealing layer 204 may have an overlay margin 402 around the treatment aperture 236, which may have an additional adhesive disposed therein. As illustrated in the example of Figure 4, the treatment aperture 236 may be an ellipse or a stadium in some embodiments. The treatment aperture 236 may have an area that is equal to about 20% to about 80% of the area of the sealing layer 204 in some examples. The treatment aperture 236 may also have an area that is equal to about 20% to about 80% of the area of a face of defined by the periphery 210 of the manifold 202. A width of about 90 millimeters to about 110 millimeters and a length of about 150 millimeters to about 160 millimeters may be suitable for some embodiments of the treatment aperture 236. For example, the width of the treatment aperture 236 may be about 100 millimeters, and the length may be about 155 millimeters. In some embodiments, a suitable width for the overlay margin 402 may be about 2 millimeters to about 3 millimeters. For example, the overlay margin 402 may be coextensive with an area defined between the treatment aperture 236 and the periphery 210, and the adhesive may secure manifold 202 to the sealing layer 204.

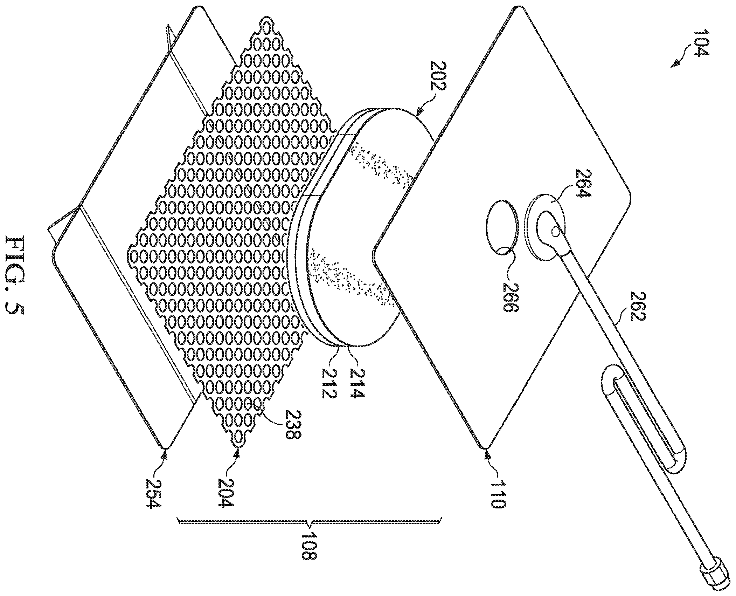

Figure 5 is an exploded view of another example of the dressing 104 of Figure 1, illustrating additional details that may be associated with some embodiments. As illustrated in Figure 5, some examples of the sealing layer 204 may not have the treatment aperture 236, and the apertures 238 may be distributed in a uniform pattern across the sealing layer 204.

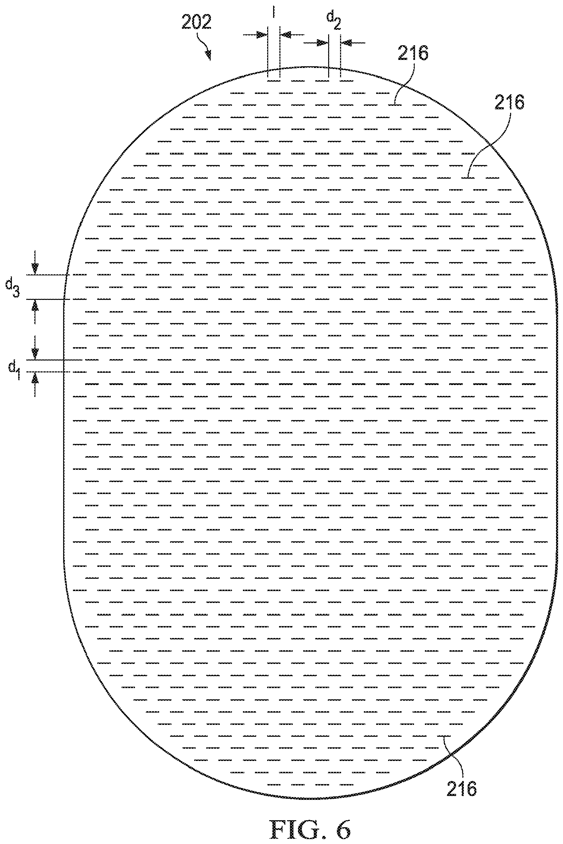

Figure 6 is a bottom view of an example of a manifold 202 that may be associated with any example embodiments of the dressing 104 or the tissue interface 108, illustrating an example pattern of perforations that may be associated with some embodiments. As illustrated in the example of Figure 6, the perforations 216 formed through the felted portion 212 or the integral film layer 226 may each consist essentially of one or more slits or slots having a length $l$. A length of about 3 millimeters may be particularly suitable for some embodiments. Figure 6 additionally illustrates an example of a uniform distribution pattern of the perforations 216. In Figure 6, the perforations 216 are substantially coextensive with the manifold 202, and are distributed across the manifold 202 in a grid of parallel rows and columns, in which the slits or slots are also mutually parallel to each other. In some embodiments, the rows may be spaced a distance $d_1$. A distance of about 3 millimeters on center may be suitable for some embodiments. The perforations 216 within each of the rows may be spaced a distance $d_2$, which may be about 3 millimeters on center in some examples. The perforations 216 in adjacent rows may be aligned or offset in some embodiments. For example, adjacent rows may be offset, as illustrated in Figure 6, so that the perforations 216 are aligned in alternating rows and separated by a distance $d_3$, which may be about 6 millimeters in some embodiments. The spacing of the perforations 216 may vary in some embodiments to increase the density of the perforations 216 according to therapeutic requirements.

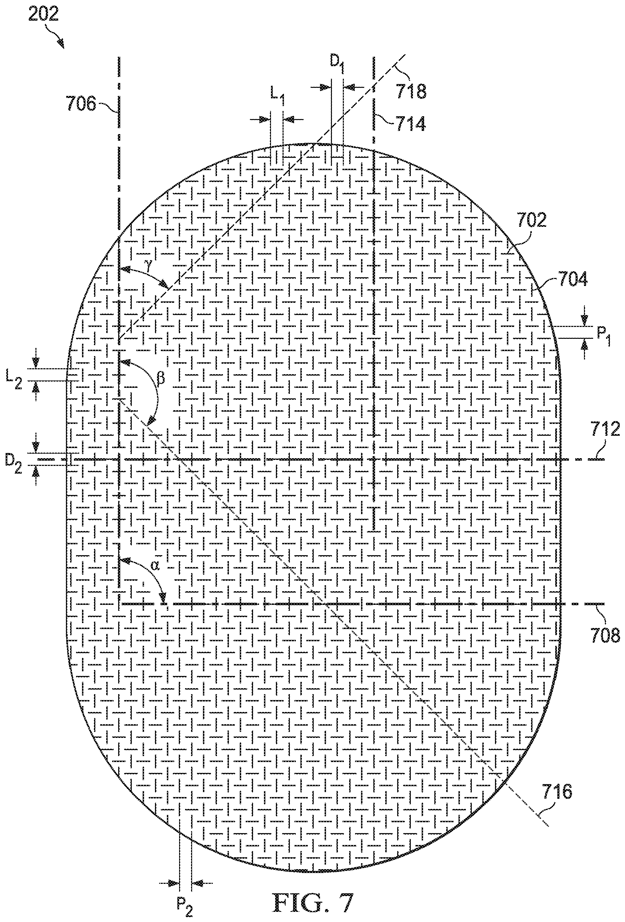

Figures 7 through 14 are bottom views of example manifolds 202 that may be associated with any example embodiments of the dressing 104 or tissue interface 108, illustrating example patterns of perforations 216 that may be associated with some example embodiments. For example, as illustrated in Figure 2, the perforations 216 formed through the felted portion 212 or the integral film layer 226 may comprise a first plurality of perforations 702 and a second plurality of perforations 704. Each of the first plurality of perforations 702 and the second plurality of perforations 704 may be linear or curved perforations, such as slots or slits. In some embodiments where the perforations are linear slots or slits, each of the first plurality of perforations 702 may have a length $L_1$ and each of the second plurality of perforations 704 may have a length $L_2$. In some embodiments, where the perforations are curved slots or slits, each of the first plurality of perforations may have a length $L_1$ measured from an end of the curved slot or slit to the other end of the curved slot or slit, and each of the second plurality of perforations may have a length $L_2$ measured from an end of the curved slot or slit to the other end of the curved slot or slit. In some embodiments, the length $L_1$ may be equal to the length $L_2$. The first plurality of perforations 702 and the second plurality of perforations 704 may be distributed across the second layer in one or more rows in one direction or in different directions.

In example embodiments, each of the first plurality of perforations 702 may have a first long axis. In some embodiments, the first long axis may be parallel to a first reference line 706 running in a first direction. In illustrative examples, each of the second plurality of perforations 704 may have a second long axis. In example embodiments, the second long axis may be parallel to a second reference line 708 running in a second direction. In some embodiments, one or both of the first reference line 706 and the second reference line 708 may be defined relative to an edge 710 or line of symmetry of the first surface 206. For example, one or both of the first reference line 706 and the second reference line 708 may be parallel or coincident with an edge 710 or line of symmetry of the first surface 206. In some illustrative embodiments, one or both of the first reference line 706 and the second reference line 708 may be rotated an angle relative to an edge 710 of the first surface 206. In example embodiments, an angle $\alpha$ may define the angle between the first reference line 706 and the second reference line 708.

In some example embodiments, the centroid of each of the first plurality of perforations 702 within a row may intersect a third reference line 712 running in a third direction. In illustrative embodiments, the centroid of each of the second plurality of perforations 704 within a row may intersect a fourth reference line 714 running in a fourth direction. In general, a centroid refers to the center of mass of a geometric object. In the case of a substantially two dimensional object such as a linear slit, the centroid of the linear slit will be the midpoint.

The pattern of perforations 216 may also be characterized by a pitch, which indicates the spacing between corresponding points on perforations 216 within a pattern. In example embodiments, pitch may indicate the spacing between the centroids of perforations 216 within the pattern. Some patterns may be characterized by a single pitch value, while others may be characterized by at least two pitch values. For example, if the spacing between centroids of the perforations 216 is the same in all orientations, the pitch may be characterized by a single value indicating the spacing between centroids in adjacent rows. In example embodiments, a pattern comprising a first plurality of perforations 702 and a second plurality of perforations 704 may be characterized by two pitch values, $P_1$ and $P_2$, where $P_1$ is the spacing between the centroids of each of the first plurality of perforations 702 in adjacent rows, and $P_2$ is the spacing between the centroids of each of the second plurality of perforations 704 in adjacent rows.

In example embodiments, within each row of the first plurality of perforations 702, each perforation may be separated from an adjacent perforation by a distance $D_1$. In some embodiments, within each row of the second plurality of perforations 704, each perforation may be separated from an adjacent perforation by a distance $D_2$. In some patterns, the rows may be staggered. The stagger may be characterized by an orientation of corresponding points in successive rows relative to an edge or other reference line associated with the first surface 206. In some embodiments, the rows of the first plurality of perforations 702 may be staggered. For example, a fifth reference line 716 in a fifth direction runs through the centroids of corresponding perforations of adjacent rows of the first plurality of perforations 702. In some example embodiments, the stagger of the rows of the first plurality of perforations 702 may be characterized by the angle $\beta$ formed between the first reference line 706 and the fifth reference line 716. In additional illustrative embodiments, the rows of the second plurality of perforations 704 may also be staggered. For example, a sixth reference line 718 in a sixth direction runs through the centroids of corresponding perforations of adjacent rows of the second plurality of perforations 704. In some embodiments, the stagger of the rows of the second plurality of perforations 704 may be characterized by the angle y formed between the first reference line 706 and the sixth reference line 718. In some example embodiments, the first surface 206 may comprise a margin having no perforations (not shown).

Figure 7 is a bottom view of another example of a manifold that may be associated with any example embodiments of the dressing 104 or the tissue interface 108, illustrating another example of a pattern that may be associated with some embodiments of the perforations 216. In the example of Figure 7, each of the first plurality of perforations 702 and the second plurality of perforations 704 may be linear slots or slits. The first reference line 706 may be parallel with an edge 710, and the second reference line 708 may be orthogonal to the edge 710. In example embodiments, the third reference line 712 is orthogonal to the first reference line 706, and the fourth reference line 714 is orthogonal to the second reference line 708. For example, the third reference line 712 may be incident with the centroids of corresponding perforations in alternating rows of the second plurality of perforations 704, and the fourth reference line 714 may intersect the centroids of corresponding perforations in alternating rows of the first plurality of perforations 702. In the example of Figure 7, the perforations 216 are arranged in a cross-pitch pattern in which each of the first plurality of perforations 702 is orthogonal along its first long axis to each of the second plurality of perforations 704 along its second long axis. For example, in Figure 7, $P_1$ is equal to $P_2$ (within acceptable manufacturing tolerances), and the cross-pitch pattern may be characterized by a single pitch value. Additionally, $L_1$ and $L_2$ may be substantially equal, and $D_1$ and $D_2$ may be also be substantially equal, all within acceptable manufacturing tolerances. The rows of the first plurality of perforations 702 and the rows of the second plurality of perforations 704 may be characterized as staggered. For example, in some example embodiments of Figure 7, $\alpha$ may be about 90°, $\beta$ may be about 135°, y may be about 45°, $P_1$ may be about 4 mm, $P_2$ may be about 4 mm, $L_1$ may be about 3 mm, $L_2$ may be about 3 mm, $D_1$ may be about 5 mm, and $D_2$ may be about 5 mm.

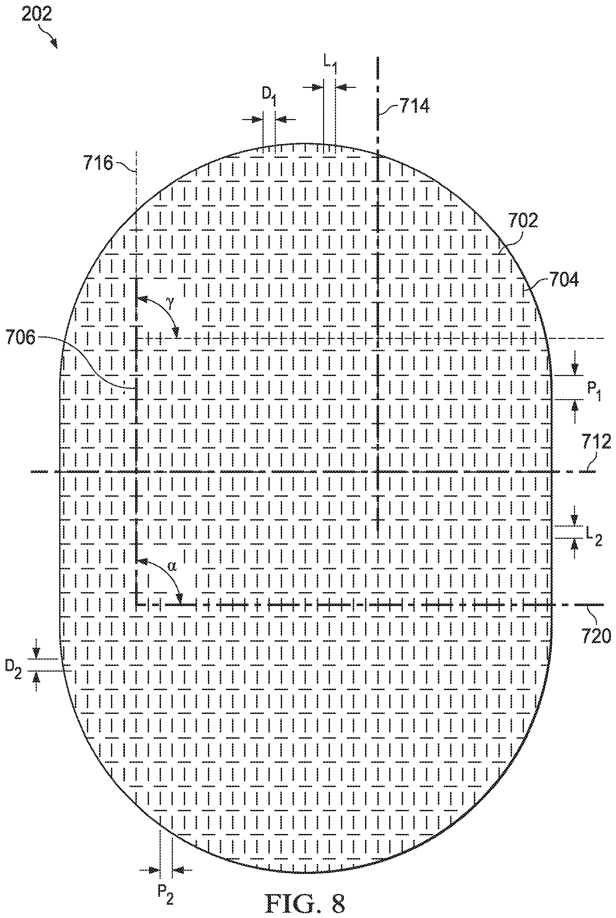

Figure 8 is a bottom view of another example of a manifold that may be associated with any example embodiments of the dressing 104 or the tissue interface 108, illustrating another example of a pattern that may be associated with some embodiments of the perforations 216. In illustrative examples of Figure 8, each of the first plurality of perforations 702 and the second plurality of perforations 704 may be linear slits or slots. The first reference line 706 may be parallel with the edge 710, and the second reference line 708 may be orthogonal to the edge 710. In some example embodiments, the third reference line 712 is orthogonal to the first reference line 706, and the fourth reference line 714 is orthogonal to the second reference line 708. In the example of Figure 8, the third reference line 712 does not intersect or touch any of the second plurality of perforations 704, and the fourth reference line 714 may intersect the centroids of corresponding perforations in alternating rows of the first plurality of perforations 702. In example embodiments, the third reference line 712 may be equidistant from the centroids of corresponding adjacent perforations within each row of the second plurality of perforations 704. The pattern of Figure 8 may also be characterized as a cross-pitch pattern, in which $P_1$ is not equal to $P_2$. In the example of Figure 8, $P_1$ is larger than $P_2$. Additionally, $L_1$, $L_2$, $D_1$, and $D_2$ are substantially equal in the example of Figure 8. In some embodiments, $\alpha$ may be about 90°, $\beta$ may be about 0° such that the first reference line 706 is coincident with the fifth reference line 716, $\gamma$ may be about 90°, $P_1$ may be about 6 mm, $P_2$ may be about 3 mm, $L_1$ may be about 3 mm, $L_2$ may be about 3 mm, $D_1$ may be about 3 mm, and $D_2$ may be about 3 mm.

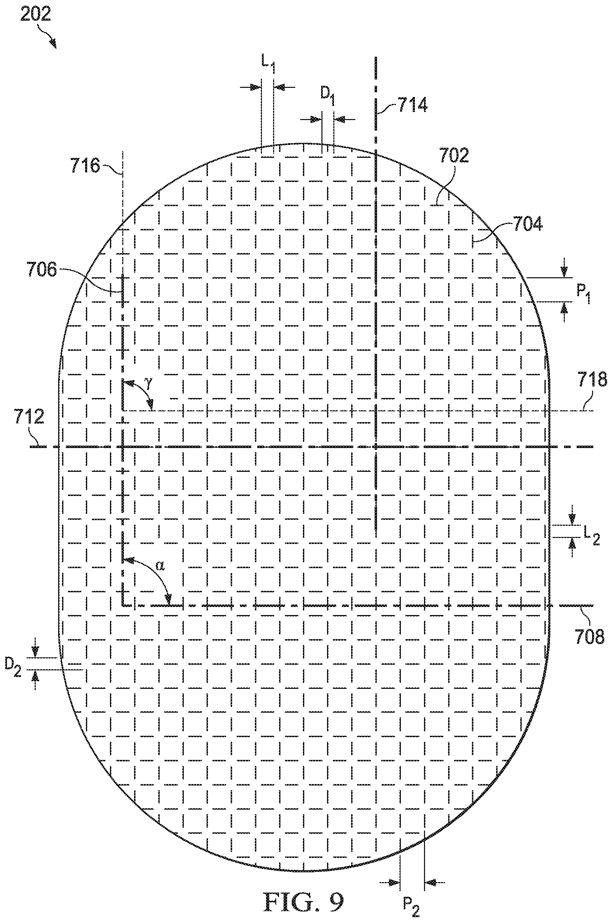

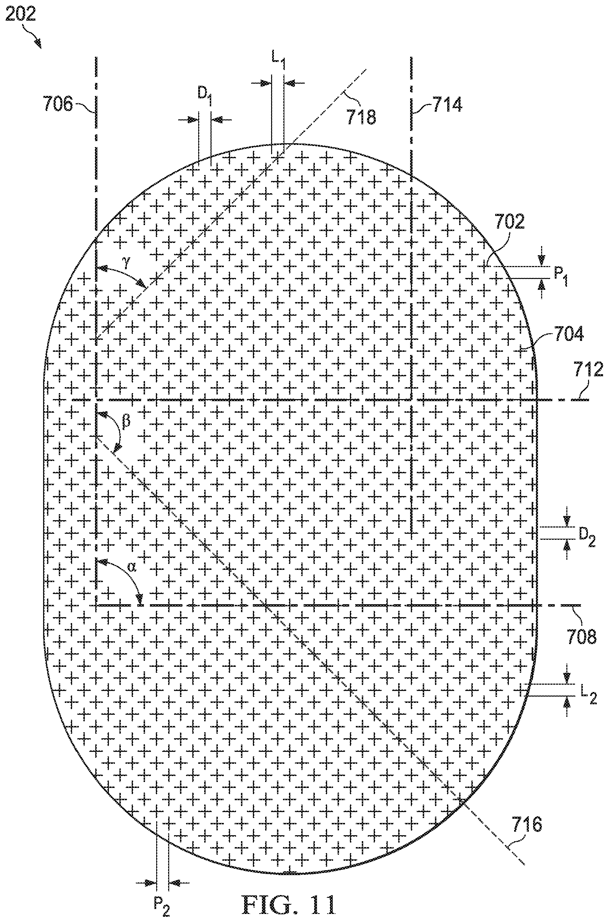

Figure 9 is a bottom view of another example of a manifold that may be associated with any example embodiments of the dressing 104 or the tissue interface 108, illustrating another example of a pattern that may be associated with some embodiments of the perforations 216. In the example of Figure 9, each of the first plurality of perforations 702 and the second plurality of perforations 704 may be linear slits or slots. The first reference line 706 may be parallel with an edge 710, and the second reference line 708 may be orthogonal to an edge 710. In example embodiments, the third reference line 712 is orthogonal to the first reference line 706, and the fourth reference line 714 is orthogonal to the second reference line 708. In the example of Figure 9, the third reference line 712 does not intersect or touch any of the second plurality of perforations 704, and the fourth reference line 714 does not intersect or touch any of the first plurality of perforations 702. In example embodiments, the third reference line 712 may be equidistant from the centroids of corresponding adjacent perforations within each row of the second plurality of perforations 704, and the fourth reference line 714 may be equidistant from the centroids of corresponding adjacent perorations within each row of the first plurality of perforations 702. The pattern of Figure 9 may be characterized as a cross-pitch pattern, in which $P_1$ is substantially

equal to $P_2$. Additionally, $L_1$, $L_2$, $D_1$, and $D_2$ are substantially equal in the example of Figure 11. In some embodiments, $\alpha$ may be about 90°, $\beta$ may be about 0° such that the first reference line 706 is coincident with the fifth reference line 716, $\gamma$ may be about 90°, $P_1$ may be about 6 mm, $P_2$ may be about 6 mm, $L_1$ may be about 3 mm, $L_2$ may be about 3 mm, $D_1$ may be about 3 mm, and $D_2$ may be about 3 mm.

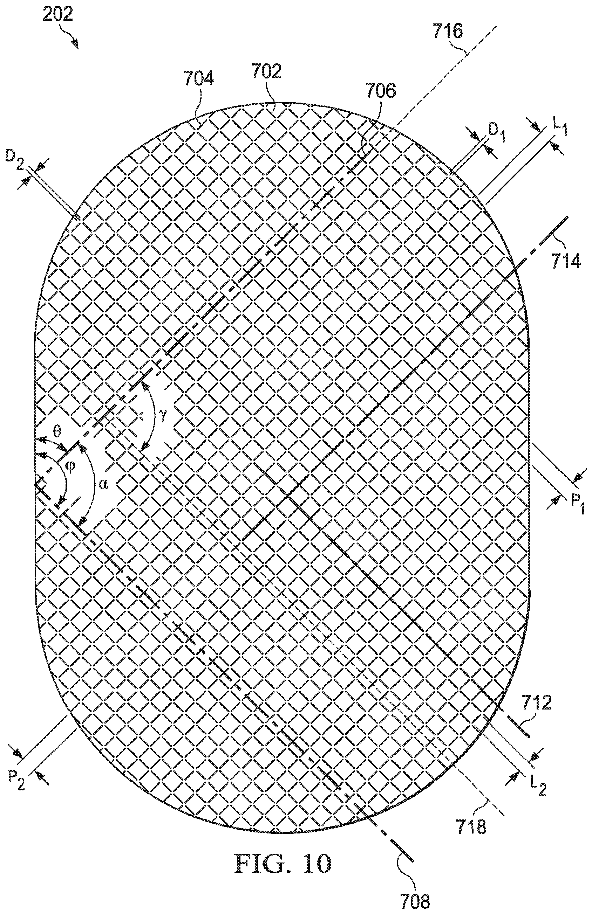

Figure 10 is a bottom view of another example of a manifold that may be associated with any example embodiments of the dressing 104 or the tissue interface 108, illustrating another example of a pattern that may be associated with some embodiments of the perforations 216. In the example of Figure 10, each of the first plurality of perforations 702 and the second plurality of perforations 704 may be linear slits or slots. The first reference line 706 may form an angle $\theta$ with an edge 710, and the second reference line 708 may form an angle $\varphi$ an edge 710. In example embodiments, the third reference line 712 is orthogonal to the first reference line 706, and the fourth reference line 714 is orthogonal to the second reference line 708. In the example of Figure 10, the third reference line 712 does not intersect or touch any of the second plurality of perforations 704, and the fourth reference line 714 does not intersect or touch any of the first plurality of perforations 702. In example embodiments, the third reference line 712 may be equidistant from the centroids of corresponding adjacent perforations within each row of the second plurality of perforations 704, and the fourth reference line 714 may be equidistant from the centroids of corresponding adjacent perforations within each row of the first plurality of perforations 702. The pattern of Figure 10 may be characterized as a cross-pitch pattern, in which $P_1$ is substantially equal to $P_2$. Additionally, $L_1$ may be substantially equal to $L_2$, and $D_1$ may be substantially equal to $D_2$ in the example of Figure 10. In some embodiments, $\beta$ may be about 0° such that the first reference line 706 is coincident with the fifth reference line 716, $\gamma$ may be about 90°, $\theta$ may be about 45°, and $\varphi$ may be about 135°.

Figure 11 is a bottom view of another example of a manifold that may be associated with any example embodiments of the dressing 104 or the tissue interface 108, illustrating another example of a pattern that may be associated with some embodiments of the perforations 216. In some embodiments of Figure 11, each of the first plurality of perforations 702 and the second plurality of perforations 704 may be linear slits or slots. The first reference line 706 may be parallel with an edge 710, and the second reference line 708 may be orthogonal to an edge 710. In example embodiments, the third reference line 712 is orthogonal to the first reference line 706, and the fourth reference line 714 is orthogonal to the second reference line 708. For example, the third reference line 712 may be incident with the centroids of corresponding perforations in alternating rows of the second plurality of perforations 704, and the fourth reference line 714 may be incident with the centroids of corresponding perforations in alternating rows of the first plurality of perforations 702. In the example of Figure 11, the centroid of each perforation of the first plurality of perforations 702 is incident with the centroid of a perforation of the second plurality of perforations 704. The perforations 216 are arranged in a cross-pitch pattern in which each of the first plurality of perforations 702 is orthogonal along its first long axis to each of the second plurality of perforations 704 along its second long axis. For example, in Figure 11, $P_1$ is substantially equal to $P_2$, and the cross-pitch pattern may be characterized by a single pitch value. Additionally, $L_1$ and $L_2$ may be substantially equal, and $D_1$ and $D_2$ may be also be substantially equal, all within acceptable manufacturing tolerances. The rows of the first plurality of perforations 702 and the rows of the second plurality of perforations 704 may be characterized as staggered. In some example embodiments of Figure 11, $\alpha$ may be about 90°, $\beta$ may be about 135°, $\gamma$ may be about 45°.

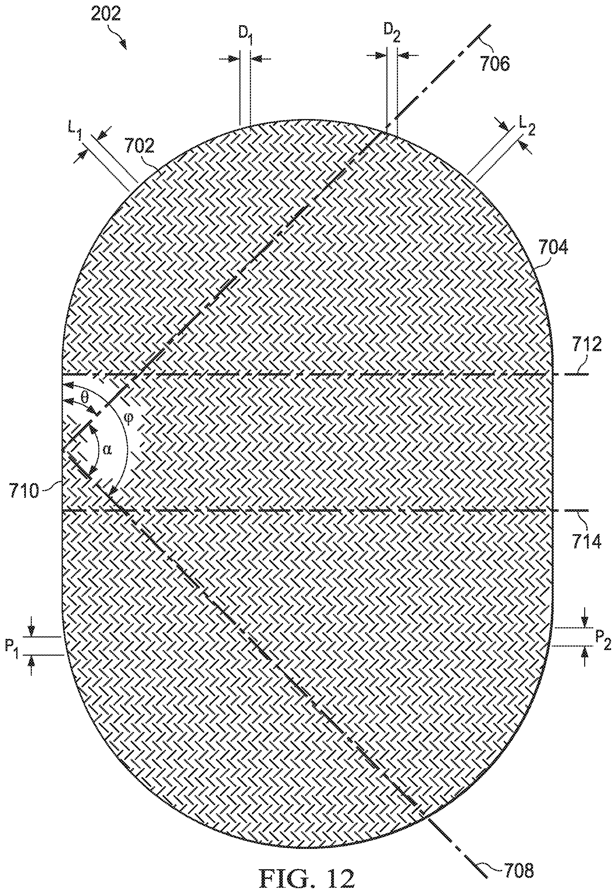

Figure 12 is a bottom view of another example of a manifold that may be associated with any example embodiments of the dressing 104 or the tissue interface 108, illustrating another example of a pattern that may be associated with some embodiments of the perforations 216. In the example of Figure 12, each of the first plurality of perforations 702 and the second plurality of perforations 704 may be linear slits or slots. The first reference line 706 may form an angle $\theta$ with an edge 710. The second reference line 708 may form an angle $\varphi$ with an edge 710. In example embodiments of Figure 12, the third reference line 712 and the fourth reference line 714 may be orthogonal to an edge 710. In the example of Figure 12, the rows of the first plurality of perforations 702 and the rows of the second plurality of perforations 704 may be characterized as mirrored rows running in one direction parallel with an edge 710 of the first surface 206. For example, $L_1$ and $L_2$ may be substantially equal, $D_1$ and $D_2$ may be substantially equal, and $P_1$ and $P_2$ may be substantially equal, within acceptable manufacturing tolerances. In some embodiments, $\theta$ may be about 45°, and $\varphi$ may be about 135°. The pattern of Figure 12 may be characterized as a herringbone pattern.

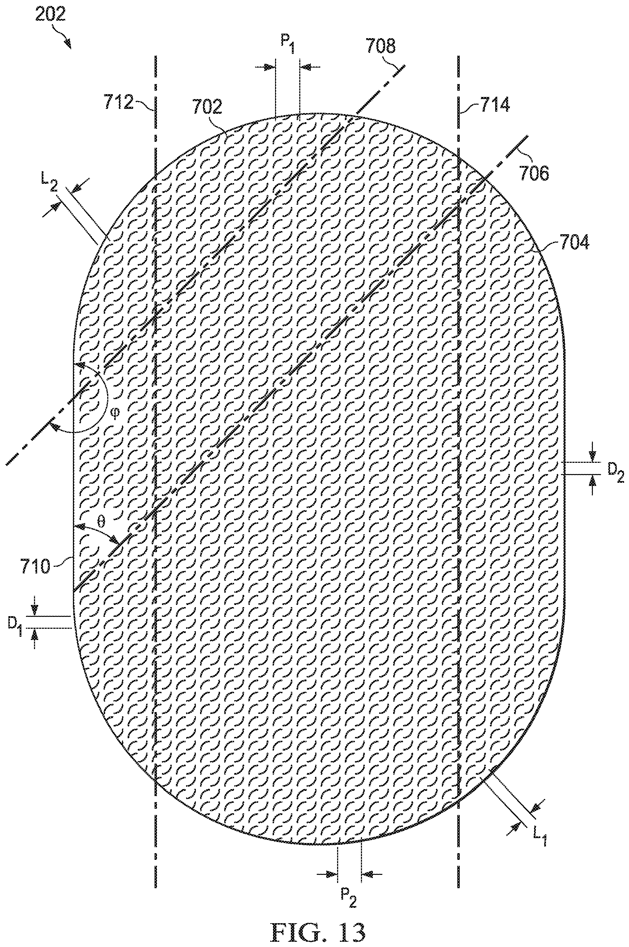

Figure 13 is a bottom view of another example of a manifold that may be associated with any example embodiments of the dressing 104 or the tissue interface 108, illustrating another example of a pattern that may be associated with some embodiments of the perforations 216. In the example of Figure 13, each of the first plurality of perforations 702 and the second plurality of perforations 704 may be curved slits or slots. The first reference line 706 may form an angle $\theta$ with an edge 710. The second reference line 708 may form an angle $\varphi$ with an edge 710. In example embodiments of Figure 13, the third reference line 712 and the fourth reference line 714 may be parallel to an edge 710. In the example of Figure 13, the rows of the first plurality of perforations 702 and the rows of the second plurality of perforations 704 may be characterized as mirrored rows running in one direction parallel with an edge 710 of the first surface 206. The rows of the first plurality of perforations 702 and the rows of the second plurality of perforations 704 may be characterized as in an embodiment of Figure 13. For example, $L_1$ and $L_2$ may be substantially equal, $D_1$ and $D_2$ may be substantially equal, and $P_1$ and $P_2$ may be substantially equal, within acceptable manufacturing tolerances. In some embodiments,

$\theta$ may be about 45°, and $\varphi$ may be about 225°.

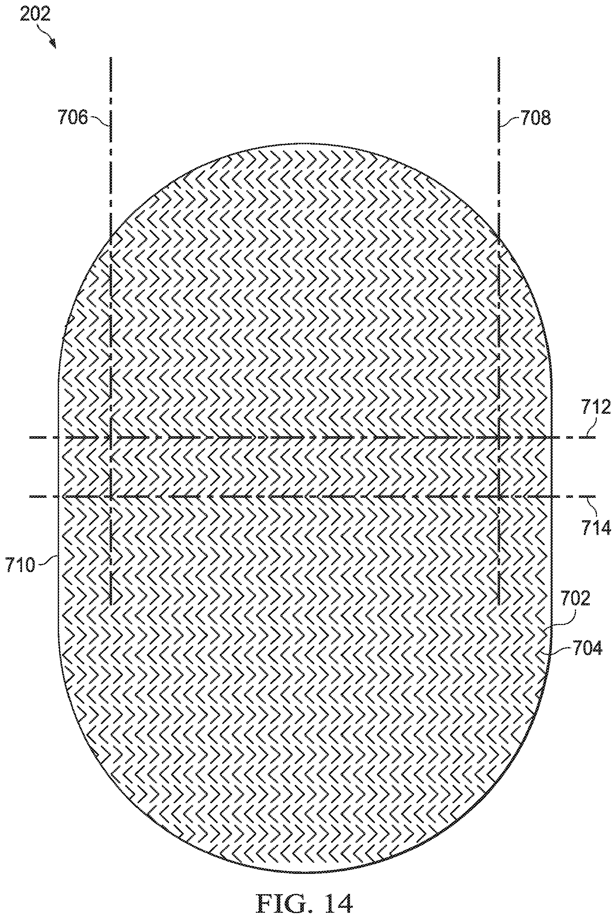

Figure 14 is a bottom view of another example of a manifold that may be associated with any example embodiments of the dressing 104 or the tissue interface 108, illustrating another example of a pattern that may be associated with some embodiments of the perforations 216. In the example of Figure 14, each of the first plurality of perforations 702 and the second plurality of perforations 704 may be characterized as chevron slits or slots. Each chevron slit or slot may be formed from two orthogonal linear slits or slots of the same length coincident at an endpoint. The chevron slit or slot may be characterized as pointing in the direction defined by the vector drawn from the centroid of the chevron slit or slot to the coincident endpoints. Within each row of the first plurality of perforations 702, the chevron slits or slots point in the same direction. Within each row of the second plurality of perforations 704, the chevron slits or slots point in the same direction. In example embodiments, the chevron slits or slots of the first plurality of perforations 702 and the chevron slits or slots of the second plurality of perforations 704 point in opposite directions. In example embodiments, the first reference line 706 and the second reference line 708 may be parallel with an edge 710. In illustrative embodiments, the third reference line 712 and the fourth reference line 714 may be orthogonal to the first reference line 706. In the example of Figure 14, the rows of the first plurality of perforations 702 and the rows of the second plurality of perforations 704 may be characterized as mirrored rows running in one direction orthogonal to an edge 710 of the first surface 206.

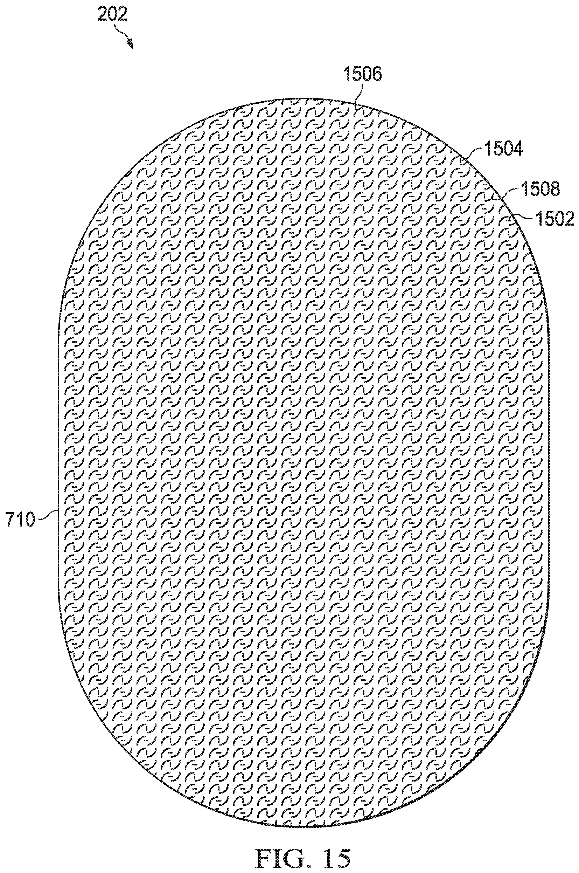

Figure 15 is a bottom view of another example of a manifold that may be associated with any example embodiments of the dressing 104 or the tissue interface 108, illustrating another example of a pattern that may be associated with some embodiments of the perforations 216. Certain patterns of the perforations 216 may comprise a third plurality of perforations 1502, a fourth plurality of perforations 1504, a fifth plurality of perforations 1506, and a sixth plurality of perforations 1508. Each of the third plurality of perforations 1502 may be a linear slit or slot substantially orthogonal along a long axis to the edge 710. Each of the fourth plurality of perforations 1504 may be a linear slit or slot substantially orthogonal to the long axis of third plurality of perforations 1502 along a long axis. Each of the fifth plurality of perforations 1506 may be a curved slit or slot with its long axis rotated to form a 45° angle with the edge 710. Each of the sixth plurality of perforations 1508 may be a curved slit or slot with its long axis rotated to form a 225° angle with the edge 710. Within each row, the pattern of perforations 216 may be a repeating pattern of one of the fifth plurality of perforations 1506, one of the third plurality of perforations 1502, one of the sixth plurality of perforations, 1520, one of the fifth plurality of perforations 1506, one of the fourth plurality of perforations 1504, and one of the sixth plurality of perforations 1508, in sequence. Each alternating row of the pattern of perforations 216 may be shifted three positions, in either direction.

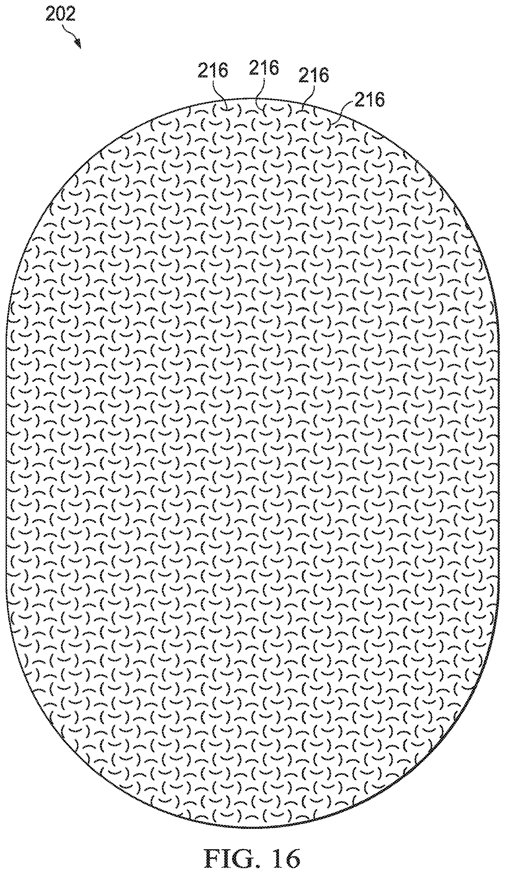

Figures 16 through 18 are bottom views of example manifolds 202 that may be associated with any example embodiments of the dressing 104 or the tissue interface 108, illustrating additional details that may be associated with some embodiments of the perforations 216. For example, as illustrated in Figure 2, the perforations 216 may be distributed across the first surface 206 in a pattern of rows. In some embodiments, each perforation 216 along a row may be rotated about 90° with respect to an adjacent perforation 216. Each perforation 216 along a row may be rotated about 90° clockwise or 90° counterclockwise with respect to a preceding adjacent perforation 216 in the row. In example embodiments of the pattern of perforations 216, every second row may be offset by one perforation 216 with respect to the previous row. The pattern of Figures 16 through 18 may be characterized as a pattern of offset rows. Example embodiments of the pattern of Figures 16 through 18 may additionally be characterized as a pattern of rotating perforations 216. In illustrative embodiments, the first surface 206 may comprise a margin having no perforations (not shown).

Figure 16 is a bottom view of another example of a manifold 202 that may be associated with any example embodiments of the dressing 104 or the tissue interface 108, illustrating example embodiments where the perforations 216 comprise curved slits or slots. In some example embodiments, the perforations 216 within a row alternate between being parallel with an edge 710 of the first surface 206 along a long axis of the perforation 216 and being orthogonal to an edge 710 of the second layer along the long axis.

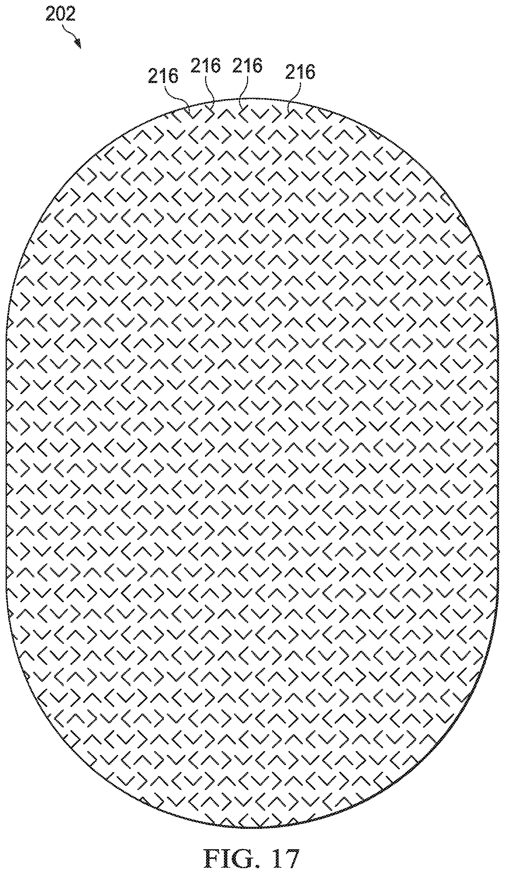

Figure 17 is a bottom view of another example of a manifold 202 that may be associated with any example embodiments of the dressing 104 or the tissue interface 108, illustrating some embodiments where the perforations 216 comprise chevron slits or slots. In some example embodiments, the perforations 216 within a row alternate between being parallel with an edge 710 of the first surface 206 along a long axis of the perforation 216 and being orthogonal to an edge 710 of the second layer along the long axis.

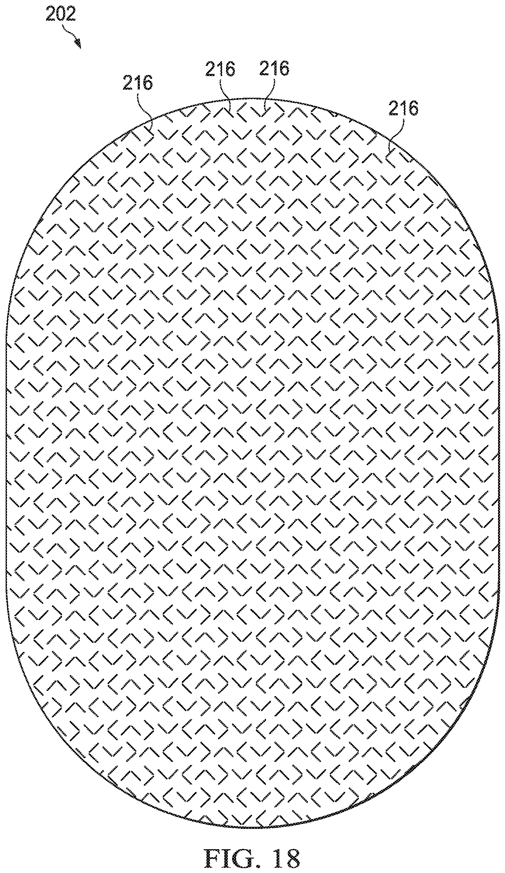

Figure 18 is a bottom view of another example of a manifold 202 that may be associated with any example embodiments of the dressing 104 or the tissue interface 108, illustrating example embodiments where the perforations 216 comprise split-chevron slits or slots. Each split-chevron slit or slot may be formed from two orthogonal non-incidental linear slits or slots mirrored about an axis bisecting the angle formed by the intersection of the orthogonal long axis of the linear slits or slots. In some example embodiments, the perforations 216 within a row alternate between being parallel with an edge 710 of the first surface 206 along a long axis of the perforation 216 and being orthogonal to an edge 710 of the second layer along the long axis.

In additional embodiments, $P_1$ may be in a range of about 4 millimeters to about 6 millimeters, $P_2$ may be in a range of about 3 mm to about 6 mm. In illustrative embodiments, $D_1$ may be in a range of about 3 mm to about 5 mm, and $D_2$ may be in a range of about 3 mm to 5 mm. In some embodiments, the margin may comprise between about 30% to about 80% of the total surface area of the first surface 206. In some embodiments, there may be an equal number of perforations 216 in the first plurality of perforations 702 as the number of perforations 216 in the second plurality of perforations 704.

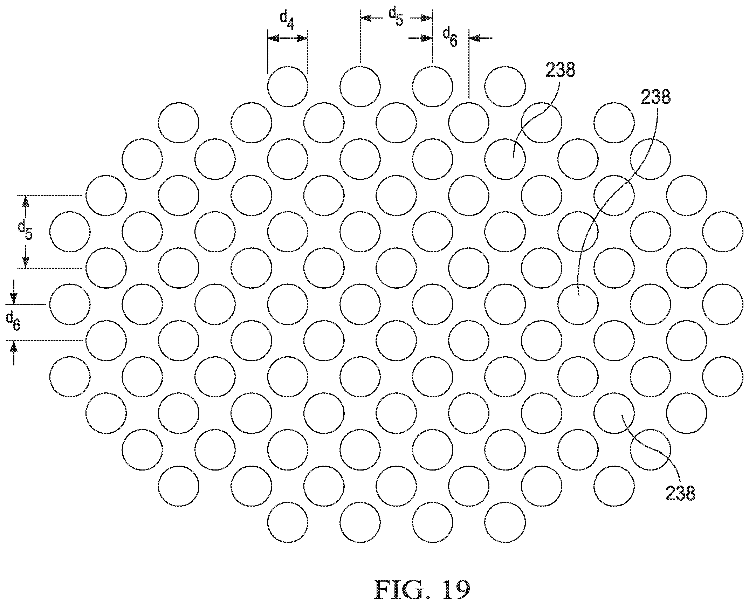

Figure 19 is a schematic view of an example configuration of apertures 238 that may be associated with some embodiments of an optional sealing layer 204. In the example of Figure 19, the apertures 238 are generally circular and have a diameter $d_4$, which may be about 6 millimeters to about 8 millimeters in some embodiments. A diameter $d_4$ of about 7 millimeters may be particularly suitable for some embodiments. Figure 19 also illustrates an example of a uniform distribution pattern of the apertures 238. In Figure 7, the apertures 238 are distributed across the sealing layer 204 in a grid of parallel rows and columns. Within each row and column, the apertures 238 may be equidistant from each other, as illustrated in the example of Figure 19. Figure 19 illustrates one example configuration that may be particularly suitable for many applications, in which the apertures 238 are spaced a distance $d_5$ apart along each row and column, with an offset of $d_6$. In some examples, the distance $d_5$ may be about 9 millimeters to about 10 millimeters, and the offset $d_6$ may be about 8 millimeters to about 9 millimeters.

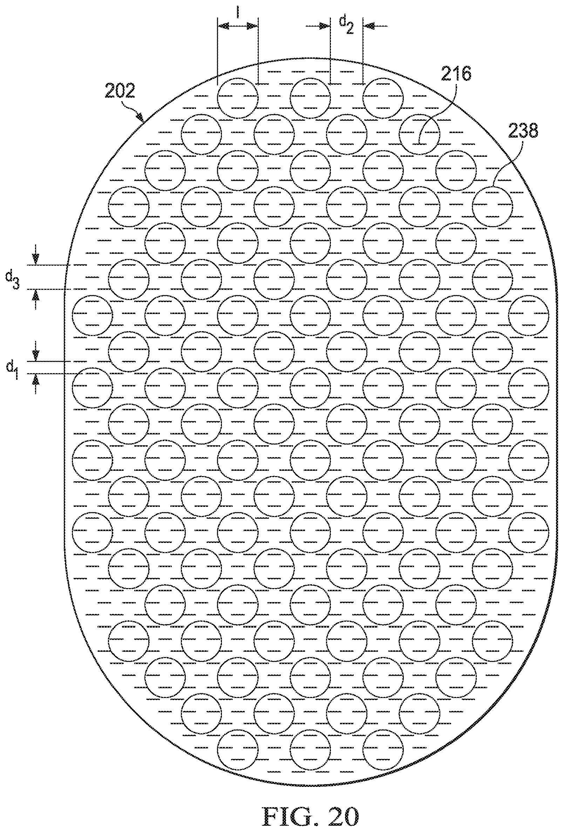

Figure 20 is a bottom view showing an example of a manifold 202 that may be associated with any example embodiments of the dressing 104 or the tissue interface 108 with the apertures 238 in the optional sealing layer 204 of Figure 19 overlaid on the manifold 202. For example, as illustrated in Figure 20, more than one of the perforations 216 may be aligned, overlapping, in registration with, or otherwise fluidly coupled to the apertures 238 in some embodiments. In some embodiments, one or more of the perforations 216 may be only partially registered with the apertures 238. The apertures 238 in the example of Figure 20 are generally sized and configured so that at least four of the perforations 216 is registered with each one of the apertures 238. In some examples, one or more of the perforations 216 may be registered with more than one of the apertures 238. For example, any one or more of the perforations 216 may be a perforation or a fenestration that extends across two or more of the apertures 238. Additionally or alternatively, one or more of the perforations 216 may not be registered with any of the apertures 238.

As illustrated in the example of Figure 20, the apertures 238 may be sized to expose a portion of the first surface 206, the perforations 216, or both through the sealing layer 204. The apertures 238 in the example of Figure 8 are generally sized to expose more than one of the perforations 216. Some or all of the apertures 238 may be sized to expose two or three of the perforations 216. In some examples, the length of each of the perforations 216 may be substantially smaller than the diameter of each of the apertures 238. More generally, the average dimensions of the perforations 216 are substantially smaller than the average dimensions of the apertures 238. In some examples, the apertures 238 may be elliptical, and the length of each of the perforations 216 may be substantially smaller than the major axis or the minor axis. In some embodiments, though, the dimensions of the perforations 216 may exceed the dimensions of the apertures 238, and the size of the apertures 238 may limit the effective size of the perforations 216 exposed to the lower surface of the dressing 104.

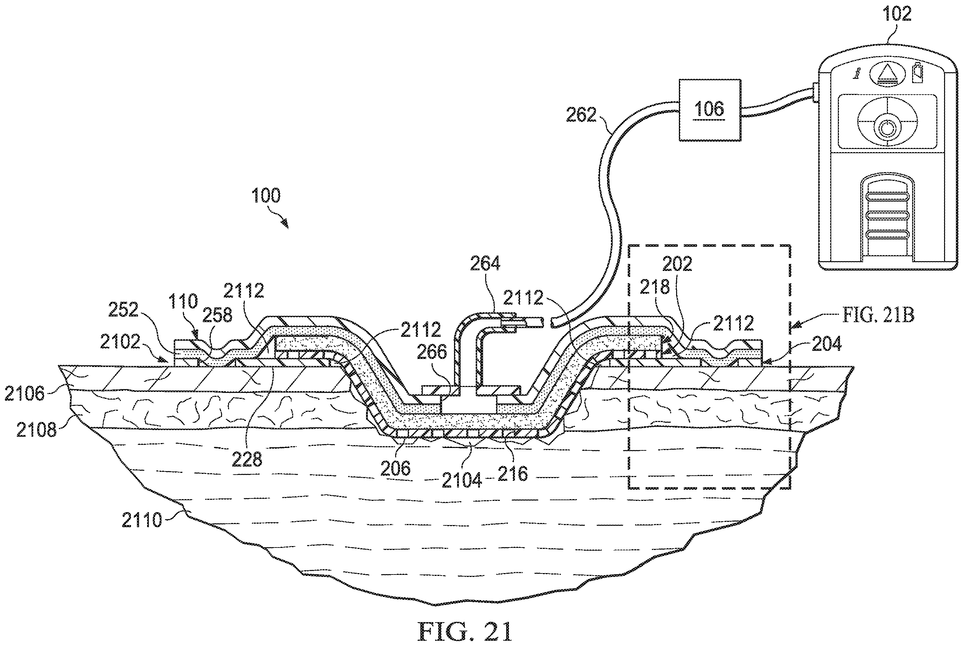

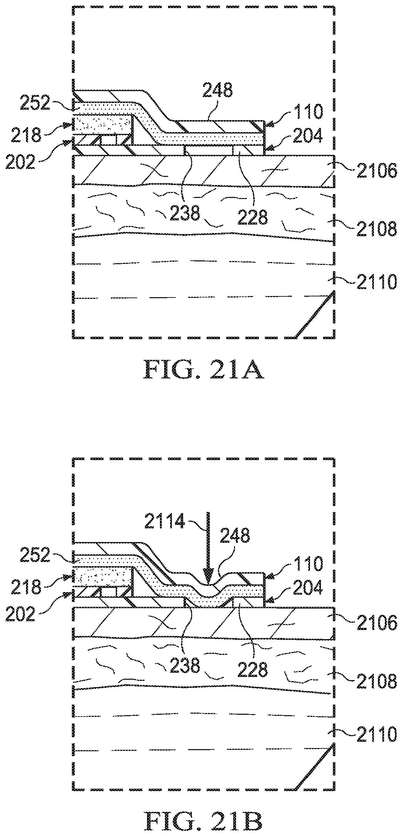

Figure 21 is a cross-sectional view of the example dressing of Figure 2E, taken at line 21-21, applied to an example tissue site 2102, and illustrating additional details associated with some examples of the therapy system 100 of Figure 1. In some embodiments, the dressing 104 may be configured to interface with a tissue site 2102. For example, the dressing 104 may be generally configured to be positioned adjacent to the tissue site 2102 and/or in contact with a portion of the tissue site 2102, substantially all of the tissue site 2102, or the tissue site 2102 in its entirety, or tissue around the tissue site 2102. In some examples, the tissue site 2102 may be or may include a defect or a targeted treatment site, such as a wound, that may be partially or completely filled or covered by the dressing 104. In various embodiments, the dressing 104 may take many forms, and may have many sizes, shapes, or thicknesses depending on a variety of factors, such as the type of treatment being implemented or the nature and size of the tissue site 2102. For example, the shape and the size of the dressing 104 may be adapted to the contours of deep and irregularly shaped tissue sites and/or may be configured to be adapted to a given shape or contour. In some embodiments, the tissue site 2102 may include a wound 2104 that extends through the epidermis and into a dermis. In some examples, as shown in Figure 21, the tissue site may include a wound which extends through the epidermis 2106, dermis 2108, and into subcutaneous tissue 1325.

As shown in Figure 21, in some embodiments, at least a portion of the first surface 206 of the manifold 202 may be placed within, over, on, against, or otherwise proximate to the wound 2104. In some examples, at least a portion of the first surface 228 of the sealing layer 204 may be placed on epidermis 2106 proximate the wound 2104 at or near the tissue site 2102. In some examples, at least a portion of the adhesive 252 may be brought into contact with the epidermis 2106 through one or more of the apertures 238. Thus, the dressing 104 may provide the sealed therapeutic environment 2112 proximate to the wound 2104. In some examples, the sealed therapeutic environment 2112 may be defined as the region of the dressing 104 between the cover 110 and the tissue site 2102 which is substantially isolated from the external environment and in fluid communication with the wound 2104. In operation, negative pressure from the negative-pressure source 102 may be provided to the wound 2104 and/or fluid may be removed from the wound 2104 through the sealed therapeutic environment 2112. For example, fluid may travel from the wound 1310 through at least one of the perforations in the manifold 202 and through the manifold 202, before being removed from the dressing 104 through the aperture 266 and the dressing interface 264. In some embodiments which include a secondary manifold 218 disposed between the manifold 202 and the cover 110, such as shown in Figure 21, the fluid removed from the wound may travel through the open pores of the secondary manifold 218 before being removed from the dressing 104 through the dressing interface 264. Fluid may be removed from the dressing through the dressing interface 264 and the fluid conductor 262, and optionally be collected within the container 106.

Figure 21A illustrates additional details that may be associated with some examples of the dressing 104. For example, the sealing layer 204 may be sufficiently tacky at the first surface 228 to hold the dressing 104 in position relative to the epidermis 2106 and wound 2104, while also allowing the dressing 104 to be removed or repositioned without trauma to the epidermis 2106, wound 2104, and/or tissue site 2102. For example, the sealing layer 204 may be formed of a silicone polyurethane material, which may form sealing couplings at the first surface 228 with the epidermis 2106. In some embodiments, the bond strength or tackiness of the sealing couplings may have a peel adhesion or resistance to being peeled from a stainless steel material between about 0.5 N/25 mm to about 1.5 N/25 mm on stainless steel substrate at 25° C at 50% relative humidity based on ASTM D3330. The sealing layer 204 may achieve this bond strength after a contact time of less than 60 seconds. Tackiness may be considered a bond strength of an adhesive after a very low contact time between the adhesive and a substrate. In example embodiments, the sealing layer 204 may have a thickness in a range of about 200 micrometers to about 1,000 micrometers. Removing the release liner 254 may also expose the adhesive 252 through the apertures 238 of the sealing layer 204. In the assembled state, the thickness of the sealing layer 204 may create a gap between the adhesive 252 and the epidermis 2106 through the apertures 238 of the sealing layer 204 such that the adhesive 252 is not in contact with the epidermis 2106.

Figure 21B is a detail view, taken at reference FIG. 21B in Figure 21, illustrating additional details that may be associated with some example embodiments of the dressing 104. Figure 21B illustrates the adhesive 252 after it has been brought into contact with the epidermis 2106 by a force 2114 applied to the second surface 248 of the cover 110 at the apertures 238. In use, if the assembled dressing 104 is in the desired location, force 2114 may be applied to the second surface 248 at the apertures 238 to cause the adhesive 252 to be pressed at least partially into contact with the epidermis 2106 to form bonding couplings. The bonding couplings may provide secure, releasable mechanical fixation of the dressing 104 to the epidermis 2106. The sealing couplings between the sealing layer 204 and the epidermis 2106 may not be as mechanically strong as the bonding couplings between the adhesive 252 and the epidermis 2106. The bonding couplings may anchor the dressing 104 to the epidermis 2106, inhibiting migration of the dressing 104.

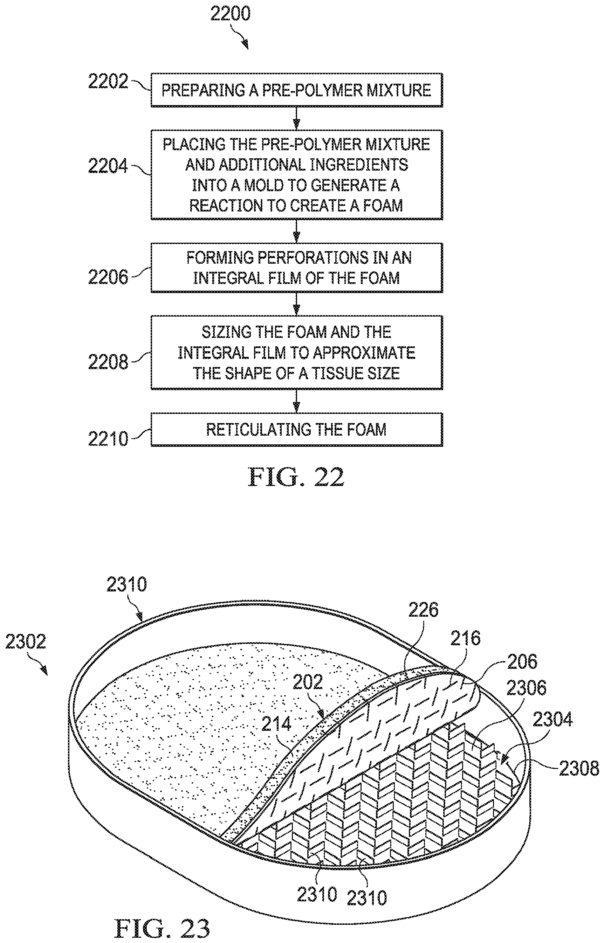

Figure 22 is a flowchart of an example method 2200 for forming some embodiments of a manifold 202 that may be associated with any examples of the dressing 104 or the tissue interface 108 in accordance to this disclosure. For example, forming the manifold 202 may begin at step 2202 with preparing a pre-polymer mixture for use in creating a foam. In some embodiments, the pre-polymer mixture may include the ingredients for forming a polyurethane foam when mixed with water. For example, the pre-polymer mixture may include ingredients for forming a polyurethane foam after the ingredients are mixed with water. For example, the pre-polymer mixture may include isocyanate and polyol. In some embodiments, the pre-polymer mixture may include ingredients for forming a polyethylene foam after the ingredients are mixed with low boiling point liquids or pressurized gasses. For example, the pre-polymer mixture may include molten polyethylene and low boiling point liquids such as fluorocarbons, pressurized gasses such as nitrogen, and/or chemical blowing agents such as citric acid and carbonate or bicarbonate salts.

The method 2200 may further include, at step 2204, placing a pre-polymer mixture and an additional ingredient (for example, water), into a mold in order to generate a reaction to create the foam. In some examples, step 2204 may include injecting the pre-polymer mixture and water into a closed mold. As the ingredients of the pre-polymer mixture react within the mold, the foam of the non-felted portion 214 of the manifold 202 may be generated. As the foam is formed, a film may also develop on one or more surfaces of the foam, forming the film layer 226 on the first surface 206 of the manifold 202. For example, as the foam contacts one or more walls of the mold, the relative temperature of the portion, or surface, of the foam contacting the wall of the mold is reduced, thereby retarding or stopping the reaction of the ingredients forming the foam. As a result, an integral film skin or film layer 226 may be formed on the surfaces of the foam contacting the wall of the mold, with the integral film skin or film layer 226 in effect being a portion of the foam having a very high density. For example, the generated integral film skin or film layer 226 may have a much higher density than the remainder of the generated foam in the non-felted-portion 214 of the manifold 202. In some embodiments, the integral film skin or film layer 226 may have a sufficiently high density such that the integral film skin or film layer 226 is essentially without pores. In some examples where the foam is a polyurethane foam, the integral film skin or film layer 226 may be in the form of a polyurethane elastomer.

The method 2200 may additionally include, at step 2206, forming the perforations 216 in the integral film skin or film layer 226 of the manifold 202. In some embodiments, the perforations 216 may also extend through the film layer 226 and into the non-felted portion 214 of the manifold 202. In some embodiments, the perforations 216 may be formed during the formation of the foam or non-felted portion 214 and the integral film skin or film layer 226 within the mold, or as a process following the formation of the foam or non-felted portion 214 and the integral film skin or film layer 226. In some embodiments, the perforations 216 may be formed by one or more projections such as pins, rods, or blades positioned within or formed as part of the mold. For example, as the pre-polymer mixture reacts to form the foam or non-felted portion 214 and the integral film skin or film layer 226, spaces within one or both of the foam or non-felted portion 214 and the integral film skin or film layer 226 may be preserved by the projections of the mold, resulting in the perforations 216 being formed. Additionally or alternatively, the perforations 216 may be formed in the integral film skin or film layer 226 and/or the foam or non-felted portion 214 after the manifold 202 is removed from the mold, such as by using one or more blades, pins, rods, or any other appropriate cutting instruments and/or mechanisms.

The method 2200 may additionally include, at step 2208, cutting or sizing the manifold 202, such as by sizing the foam or non-felted portion 214 and the integral film skin or film layer 226 to approximate the size and/or shape of a tissue site. In some examples, it may be appropriate or beneficial to size the manifold 202 such that it extends past the perimeter of the wound to cover the periwound area.

The method 2200 may further include, at step 2210, reticulating the foam portion or the non-felted portion 214 of the manifold 202. In some embodiments, the reticulation process may be performed after the foam or non-felted portion 214 and the integral film skin or film layer 226 have been formed in the mold and the perforations 216 have been formed in the integral film skin or film layer 226. In some embodiments, the foam or non-felted portion 214 may be reticulated after being removed from the mold. Additionally or alternatively, the manifold 202 may be removed from the mold and stacked within a reticulation chamber where the reticulation process may be performed. In some embodiments, the perforations 216 formed in the integral film skin or film layer 226 may allow the reticulation gas to enter the pore structure of the foam or non-felted portion 214 and permit the escape of the combustion gases, such as water vapor, from the foam during the reticulation process. Additionally or alternatively, one or more cell openers may be used for creating the porosity in the foam or non-felted portion 214. For example, one or more cell openers may be used to introduce weaknesses into the cell walls of the polymer foam during its formation. Example cell openers may include calcium carbonate, nanoparticles, and/or anti-foaming agents such as siloxanes and polyethylene oxides.

In some illustrative embodiments, for example, in examples where the perforations 216 may extend through the integral film skin or film layer 226 and the foam or non-felted portion 214 from the first surface 206 through the body of the manifold 202 and through the second surface 208, the perforations 216 may act as manifolding passageways through the body of the manifold 202. Accordingly, in some examples, the step of reticulating the foam portion may not be necessary, as the channels formed through the body of the manifold 202 by the perforations 216 may enable a non-reticulated foam to have sufficient capacity for fluid handling and communication of air and fluids in negative-pressure therapy applications.

Additionally or alternatively, various other molding techniques may be used to form one or more embodiments of the manifold 202. For example, in some embodiments, foam-slab casting methods involving a foam mixture being poured into a tray may be used to create a foam for the manifold 202 in the form of a relatively thin foam sheet with an integral film or skin for the film layer 226. For example, once poured into the mold, the pre-polymer mixture and water may generate a relatively thin foam sheet with an integral film or film layer 226 formed on the first surface 206 which is in contact with the bottom of the tray mold. During formation of the foam layer, the surface of the manifold 202 in contact with the atmosphere, such as the second surface 208, may be skived to remove any intermittent-density or variable-density foam, with the remaining portion of the manifold 202 being the non-felted portion 214.

In some embodiments of the method 2200, additional steps may include forming one or more surface features on the first surface 206 of the manifold, such as on the integral film skin or film layer 226. For example, some embodiments may include using in-mold decoration techniques for providing additional or different layers or different types of film, colored or patterned features, or surface-textured features to an outer surface of the integral film skin or film layer 226. For example, such steps for creating these one or more surface features may include placing an additional textured film layer into the mold prior to the injection of the pre-polymer mixture into the mold. In some examples, prior to the injection of the pre-polymer mixture into the mold, an additional material may be spray-coated into the mold and allowed to form and cure into a film. For example, a UV-curable precursor may be spray-coated into the mold and cured into a film by UV-light crosslinking the polymer chains of the precursor.

Figure 23 is an isometric view of an example of a manifold 202 positioned within a mold 2302 during formation of a film layer 226 and a non-felted portion 214, according to some illustrative embodiments. In some embodiments, as shown in Figure 23, the mold 2302 may be an open mold, such as a tray mold. The mold 2302 may include a base portion 2304 having a first surface 2306 configured to face the first surface 206 of the manifold 202, and a periphery 2308. The mold 2302 may further include a wall or plurality of walls 2310 surrounding the base portion 2304 around the periphery 2308. The periphery 2308 and the walls 2310 may define a variety of shapes and sizes. For example, as shown in Figure 23, the periphery 2308 and the walls 2310 defines a stadium shape, or a two-dimensional shape constructed of a rectangle with semicircles at opposing ends of the rectangle. In other examples, the periphery 2308 and the walls 2310 may define an ellipse, a circle, a polygon shape, or any other suitable shape for forming a manifold 202. The mold 2302 may further include a plurality of projections 2312 formed on the first surface 2306 and extending away from the first surface 2306. The profile of the projections 2312 in a plane coplanar with the first surface 2306 may be defined by or coextensive with any of the patterns of the perforations 216, the first plurality of perforations 702, the second plurality of perforations 704, the third plurality of perforations 1502, the fourth plurality of perforations 1504, the fifth plurality of perforations 1506, and/or the sixth plurality of perforations 1508 as previously described and as shown in Figures 6-18.

The plurality of projections 2312 may occupy space within the volume of the mold 2302 such that the pre-polymer mixture does not occupy the space occupied by the projections 2312 during the formation of the manifold 202. When the manifold 202 is formed and removed from the mold 2302, the space in the film layer 226 and/or the non-felted portion 214 previously occupied by the projections 2312 will not have polymer material, and will define the perforations 216, the first plurality of perforations 702, the second plurality of perforations 704, the third plurality of perforations 1502, the fourth plurality of perforations 1504, the fifth plurality of perforations 1506, and/or the sixth plurality of perforations 1508.

Some embodiments of the manifold 202 may also be formed using other methods and manufacturing processes. For example, in some embodiments, the manifold 202 may be formed using an extrusion process. For example, the foam material forming the non-felted portion 214 may be extruded with an outer integral film skin forming the film layer 226 included on the first surface 206 of the manifold 202. Once the extrusion process is complete, the perforations 216, the first plurality of perforations 702, the second plurality of perforations 704, the third plurality of perforations 1502, the fourth plurality of perforations 1504, the fifth plurality of perforations 1506, and/or the sixth plurality of perforations 1508 may be formed through the extruded film layer 226.

The systems, apparatuses, and methods described herein may provide significant advantages. For example, by providing a felted surface with relatively small pores at the first surface 206 of the manifold 202, granulation tissue in-growth may be substantially reduced or prevented, allowing the dressing 104 to remain in place at the tissue site 2102 for a longer period of time without experiencing tissue in-growth into the manifold 202. Similarly, by providing a substantially non-porous film surface at the first surface 206 of the manifold 202, granulation tissue in-growth may be substantially reduced or prevented, allowing the dressing 104 to remain in place at the tissue site 2102 for a longer period of time without experiencing tissue in-growth into the manifold 202. Furthermore, by providing a felted surface or an integrally formed film surface at the first surface 206 of the manifold 202, adhesive may not be required in the manufacture of the manifold 202, increasing the biocompatibility of the manifold 202 and reducing material costs.

Additionally or alternatively, some embodiments of the pattern of perforations 216, the first plurality of perforations 702, the second plurality of perforations 704, the third plurality of perforations 1502, the fourth plurality of perforations 1504, the fifth plurality of perforations 1506, and/or the sixth plurality of perforations 1508 may permit the manifold 202 and/or the dressing 104 to deform to a greater degree while simultaneously conforming more closely over tissue sites with large surface areas and/or complex contours. For example, by providing perforations oriented in different directions, such as in patterns of orthogonal perforations, the overall flexibility of the manifold 202 and the dressing 104 may be increased. For example, by facilitating even expansion profiles and forces in more than one direction, the manifold 202 and the dressing 104 may deform and apply forces in a uniform manner radially. This pay permit the dressing 104 to better manage deeper wounds and large complex wounds, such as the treatment of wound with a diameter which may be greater than about 1.3 times the depth of the wound, and may allow for more extension of the dressing when applied to deeper wounds.

Additionally or alternatively, for example, as previously described, some embodiments of the sealing layer 204 may be sufficiently tacky to form sealing couplings with the epidermis 2106 to prevent dressing leaks, minimize or reduce pain upon removal of the dressing 104, and permit a two-stage application of the dressing 104. For example, the dressing 104 may be initially positioned on the epidermis 2106 without any of the adhesive 252 being in contact with the epidermis 2106. The sealing couplings may be sufficiently tacky to prevent migration of the dressing 104 relative to the epidermis 2106, but also permit the dressing 104 to be lifted from the epidermis 2106 and repositioned without any pain or damage to the epidermis 2106 and/or the tissue site 2102. Once the position of the dressing 104 has been adjusted, the adhesive 252 may be pushed through the apertures 238 by force 2114, forming stronger bonding couplings with the epidermis 2106, more securely coupling the dressing 104 to the epidermis 2106 and/or the tissue site 2102. Furthermore, by minimizing the surface area of the bonding couplings formed by the adhesive 252 with the epidermis 2106, the use of sealing couplings in conjunction with bonding couplings may facilitate an easier removal process, minimizing or reducing pain upon removal of the dressing 104. Furthermore, by providing thinner areas of the dressing 104 where only the adhesive 252 and the cover 110 are present, the MVTR of the dressing may be increased in those areas relative to the areas where the sealing layer 204 is present, and the overall MVTR of the dressing 104 as a whole may be increased.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or eliminated in various configurations for purposes of sale, manufacture, assembly, or use. For example, in some configurations that dressing 104, container 106, or both may be eliminated or separated from other components for manufacture or sale. In other example configurations, the controller 112 may also be manufactured, configured, assembled, or sold independently of other components.

**Claims**

1. A dressing for treating a tissue site with negative pressure, comprising:

an open-cell foam member (202) having a felted portion (212) and a non-felted portion (214), the felted portion (212) extending a depth into a tissue-contact surface (206) of the foam member (202); a sealing layer (204) comprising a treatment aperture (236) and a first plurality of perforations (238) around the treatment aperture (236), the felted portion (212) configured to contact the tissue site through the treatment aperture (236); a second plurality of perforations (216) formed through the felted portion (212), at least one of the second plurality of perforations (216 exposed to the tissue site through the treatment aperture (236); and a cover (110) comprising a film and an adhesive, the film disposed over the foam member (202) and coupled to the sealing layer (204) around the foam member (202), the adhesive disposed adjacent to the second plurality of perforations (216).

2. The dressing of claim 1, wherein the non-felted portion (214) has a density in a range of 20.8kg/m$^3$ (1.3 lb/ft$^3$) to 25.6 kg/m$^3$ (1.6 lb/ft$^3$).

3. The dressing of any of claims 1-2, wherein the felted portion (212) has a density in a range of about 41.6kg/m$^3$ (2.6 lb/ft$^3$) to about 256.3kg/m$^3$ (16 lb/ft$^3$).

4. The dressing of any of claims 1-3, wherein the non-felted portion (214) has a 25% compression load deflection of about 2.4kPa (0.35 pounds per square inch).

5. The dressing of any of claims 1-4, where the felted portion (212) has a 25% compression load deflection of about 4.8kPa (0.7 pounds per square inch) to about 24.1 kPa (3.5 pounds per square inch).

6. The dressing of any of claims 1-5, wherein the non-felted portion (214) has a 65% compression load deflection of about 3.0 kPa (0.43 pounds per square inch).

7. The dressing of any of claims 1-5, wherein the felted portion (212) has a 65% compression load deflection in a range of about 5.9 kPa (0.86 pounds per square inch) to about 29.6 kPa (4.3 pounds per square inch).

8. The dressing of any of claims 1-7, wherein each perforation of the second plurality of perforations (216) is a slot formed through the felted portion (212).

9. The dressing of any of claims 1-8, further comprising a third plurality of perforations formed through the felted portion (212).

10. The dressing of claim 9, wherein each perforation of the third plurality of perforations is a slot formed through the felted portion (212).

**Patentansprüche**

1. Ein Verband zum Behandeln einer Gewebestelle mit Unterdruck, aufweisend:

ein offenzelliges Schaumstoffelement (202), das über einen Filzabschnitt (212) und einen filzlosen Abschnitt (214) verfügt, wobei sich der Filzabschnitt (212) eine Tiefe in eine Gewebeberührungsoberfläche (206) des Schaumstoffelements (202) erstreckt; eine Versiegelungsschicht (204), aufweisend eine Behandlungsapertur (236) und eine erste Mehrzahl von Perforationen (238) um die Behandlungsapertur (236), wobei der Filzabschnitt (212) konfiguriert ist, um durch die Behandlungsapertur (236) die Gewebestelle zu berühren; eine zweite Mehrzahl von Perforationen (216), die durch den Filzabschnitt (212) ausgebildet sind, wobei mindestens eine der zweiten Vielzahl von Perforationen (216) durch die Behandlungsapertur (236) der Gewebestelle ausgesetzt ist; und

eine Abdeckung (110), aufweisend eine Folie und einen Kleber, wobei die Folie über dem Schaumstoffelement (202) angeordnet und mit der Versiegelungsschicht (204) um das Schaumstoffelement (202) gekoppelt ist, wobei der Kleber angrenzend an die zweite Mehrzahl von Perforationen (216) angeordnet ist.

2. Der Verband nach Anspruch 1, wobei der filzlose Abschnitt (214) über eine Dichte in einem Bereich von 20,8 kg/m$^3$ (1,3 lb/ft$^3$) bis 25,6 kg/m$^3$ (1,6 lb/ft$^3$) verfügt.

3. Der Verband nach einem der Ansprüche 1 bis 2, wobei der Filzabschnitt (212) über eine Dichte in einem Bereich von etwa 41,6 kg/m$^3$ (2,6 lb/ft$^3$) bis etwa 256,3 kg/m$^3$ (16 lb/ft$^3$) verfügt.

4. Der Verband nach einem der Ansprüche 1 bis 3, wobei der filzlose Abschnitt (214) über eine 25 %-ige Kompressionsdruckdurchbiegung von etwa 2,4 kPa (0,35 Pfund pro Quadratzoll) verfügt.

5. Der Verband nach einem der Ansprüche 1 bis 4, wobei der Filzabschnitt (212) über eine 25 % Kompressionsdruckdurchbiegung von etwa 4,8 kPa (0,7 Pfund pro Quadratzoll) bis etwa 24,1 kPa (3,5 Pfund pro Quadratzoll) verfügt.

6. Der Verband nach einem der Ansprüche 1 bis 5, wobei der filzlose Abschnitt (214) über eine 65 %-ige Kompressionsdruckdurchbiegung von etwa 3,0 kPa (0,43 Pfund pro Quadratzoll) verfügt.

7. Der Verband nach einem der Ansprüche 1 bis 5, wobei der Filzabschnitt (212) über eine 65 % Kompressionsdruckdurchbiegung in einem Bereich von etwa 5,9 kPa (0,86 Pfund pro Quadratzoll) bis etwa 29,6 kPa (4,3 Pfund pro Quadratzoll) verfügt.

8. Der Verband nach einem der Ansprüche 1 bis 7, wobei jede Perforation der zweiten Mehrzahl von Perforationen (216) ein Schlitz ist, der durch den Filzabschnitt (212) ausgebildet ist.

9. Der Verband nach einem der Ansprüche 1 bis 8, ferner aufweisend eine dritte Mehrzahl von Perforationen, die durch den Filzabschnitt (212) ausgebildet sind.

10. Der Verband nach Anspruch 9, wobei jede Perforation der dritten Mehrzahl von Perforationen ein Schlitz ist, der durch den Filzabschnitt (212) ausgebildet ist.

**Revendications**

1. Pansement permettant de traiter un site tissulaire par pression négative, comprenant :

un élément en mousse à cellules ouvertes (202) ayant une partie feutrée (212) et une partie non feutrée (214), la partie feutrée (212) s'étendant en profondeur dans une surface (206) en contact avec le tissu de l'élément en mousse (202) ;
une couche d'étanchéité (204) comprenant une ouverture de traitement (236) et une première pluralité de perforations (238) autour de l'ouverture de traitement (236), la partie feutrée (212) étant conçue pour entrer en contact avec le site tissulaire à travers l'ouverture de traitement (236) ;
une deuxième pluralité de perforations (216) formées à travers la partie feutrée (212), au moins une de la deuxième pluralité de perforations (216) étant exposée au site tissulaire à travers l'ouverture de traitement (236) ; et
un élément de recouvrement (110) comprenant un film et un adhésif, le film étant disposé sur l'élément en mousse (202) et accouplé à la couche d'étanchéité (204) autour de l'élément en mousse (202), l'adhésif étant disposé à côté de la deuxième pluralité de perforations (216).

2. Pansement selon la revendication 1, dans lequel la partie non feutrée (214) a une masse volumique comprise dans une plage de 20,8 kg/m$^3$ (1,3 lb/ft$^3$) à 25,6 kg/m$^3$ (1,6 lb/ft$^3$).

3. Pansement selon l'une quelconque des revendications 1 à 2, dans lequel la partie feutrée (212) a une masse volumique comprise dans une plage d'environ 41,6 kg/m$^3$ (2,6 lb/ft$^3$) à environ 256,3 kg/m$^3$ (16 lb/ft$^3$).

4. Pansement selon l'une quelconque des revendications 1 à 3, dans lequel la partie non feutrée (214) a une déflexion à 25 % de charge de compression d'environ 2,4 kPa (0,35 livre par pouce carré).

5. Pansement selon l'une quelconque des revendications 1 à 4, où la partie feutrée (212) a une déflexion à 25 % de charge de compression d'environ 4,8 kPa (0,7 livre par pouce carré) à environ 24,1 kPa (3,5 livres par pouce carré).

6. Pansement selon l'une quelconque des revendications 1 à 5, dans lequel la partie non feutrée (214) a une déflexion à 65 % de charge de compression d'environ 3,0 kPa (0,43 livre par pouce carré).

7. Pansement selon l'une quelconque des revendications 1 à 5, dans lequel la partie feutrée (212) a une déflexion à 65 % de charge de compression comprise dans une plage d'environ 5,9 kPa (0,86 livre par pouce carré) à environ 29,6 kPa (4,3 livres par pouce carré).

8. Pansement selon l'une quelconque des revendications 1 à 7, dans lequel chaque perforation de la deuxième pluralité de perforations (216) est une fente formée à travers la partie feutrée (212).

9. Pansement selon l'une quelconque des revendications 1 à 8, comprenant en outre une troisième pluralité de perforations formées à travers la partie feutrée (212).

10. Pansement selon la revendication 9, dans lequel chaque perforation de la troisième pluralité de perforations est une fente formée à travers la partie feutrée (212).

FIG. 1

FIG. 2A

FIG. 2B

FIG. 2C

FIG. 2D

FIG. 2E

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 21B

FIG. 21A

FIG. 21B

2200

2202 — PREPARING A PRE-POLYMER MIXTURE

2204 — PLACING THE PRE-POLYMER MIXTURE AND ADDITIONAL INGREDIENTS INTO A MOLD TO GENERATE A REACTION TO CREATE A FOAM

2206 — FORMING PERFORATIONS IN AN INTEGRAL FILM OF THE FOAM

2208 — SIZING THE FOAM AND THE INTEGRAL FILM TO APPROXIMATE THE SHAPE OF A TISSUE SIZE

2210 — RETICULATING THE FOAM

FIG. 22

FIG. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020081322 A1